# FASCICULE DE BREVET EUROPEEN

(11) **EP 1 429 803 B1**
(45) Date de publication et mention de la délivrance du brevet: **01.02.2012**
(21) Numéro de dépôt: 02772497.0
(22) Date de dépôt: 09.08.2002
(51) Int. Cl.: A61K 39/215, C07K 14/165, C12N 15/50, C12N 15/62, G01N 33/68

(54) **VACCIN ANTI-CORONAVIRUS.**
ANTI-CORONAVIRUS IMPFSTOFF
ANTI-CORONAVIRUS VACCINE

(30) Priorité: 09.08.2001 FR 0110644
(43) Date de publication de la demande: 23.06.2004
(73) Titulaire: VIRBAC, 06516 Carros (FR); INSTITUT NATIONAL DE LA RECHERCHE AGRONOMIQUE (INRA), 75341 Paris Cedex 07 (FR); Ecole Nationale Vétérinaire de Maisons ALFORT ENVA, 94704 Maisons Alfort (FR)
(72) Inventeur: AUBERT, André, F-06600 Antibes Juan les Pins (FR); DUQUESNE, Véronique, F-06510 Carros (FR); ELOIT, Marc, F-94100 Saint-Maur (FR); GONON, Valérie, F-78610 Le Perray en Yvelines (FR)
(74) Mandataire: Vialle-Presles, Marie José
(86) Numéro de dépôt international: PCT/FR2002/002843
(87) Numéro de publication internationale: WO 2003/013599

(56) Documents cités:
- WO-A-93/23421
- WO-A-93/23423
- WO-A-96/06934
- GONON V ET AL: "Clearance of infection in cats naturally infected with feline coronaviruses is associated with an anti-S glycoprotein antibody response" J GEN VIROL, vol. 80, no. 9, septembre 1999 (1999-09), pages 2315-2317, XP002200063 cité dans la demande

## Description

La présente invention est relative à un vaccin contre la péritonite infectieuse féline (PIF ou FIP pour *feline infectious peritonitis*), comprenant des peptides immunogènes inclus dans la protéine S du virus de la peritonite infectieuse féline (VPIF) qui n'induisent pas de phénomène de facilitation.

La péritonite infectieuse féline est une maladie systémique, la plupart du temps mortelle, chez les chats sauvages et domestiques. L'agent responsable est un coronavirus, le virus de la péritonite infectieuse féline (VPIF ou FIPV pour *Feline Infectious Peritonitis Virus*), qui appartient au groupe antigénique qui comprend notamment le coronavirus entérique félin (FECV), le coronavirus canin (CCV), le coronavirus de la gastro-entérite transmissible de porc (TGEV), le coronavirus respiratoire porcin (PRCV) et le coronavirus humain (HCV) qui induit, d'une façon hôte-dépendante, un éventail de symptômes qui vont de l'entérite légère à la maladie débilitante grave, et dans certains cas, jusqu'à la mort.

Le coronavirus félin, comme d'autres coronavirus, est un virus à ARN monocaténaire, de polarité positive, d'approximativement 30 kilobases. Ce virus est enveloppé et comprend des structures péplomériques dénommées "Spike", spicules ou protéine S. L'extrémité 3' de l'ARN code notamment pour les protéines structurales suivantes :
- une protéine membranaire, également dénommée protéine de matrice (M). La protéine M est la glycoprotéine membranaire la plus abondante (25-30 kDa). Seulement 10 % de la partie N-terminale de la molécule est exposée à la surface du virion. Elle semble être importante pour la maturation virale des coronavirus et pour la détermination du site au niveau duquel les particules virales sont assemblées.
- une protéine de nucléocapside (N). La protéine N, qui est également une glycoprotéine (45-50 kDa), est la plus conservée parmi les protéines structurales des coronavirus et elle est nécessaire pour encapsider l'ARN génomique et en particulier pour diriger son incorporation dans la capside. Cette protéine est vraisemblablement impliquée dans la réplication de l'ARN.
- une autre protéine membranaire, la protéine sM (*small membrane*) est également connue sous le nom de protéine E (enveloppe) (environ 10 kDa). Il s'agit d'une protéine trans-membranaire qui, comme la protéine M, joue un rôle crucial en formant une matrice intégrale pour l'architecture sphérique du virion et son bourgeonnement et
- la protéine S précitée, qui est également une protéine membranaire, qui se présente sous la forme de massues ou "Spike" ou encore spicules (S) et est également désignée sous le nom de protéine du péplomère. La protéine S est une glycoprotéine (200-220 kDa), dont une partie importante (spicules) est située à l'extérieur de l'enveloppe virale. Elle est responsable de l'attachement du virus aux récepteurs de la cellule hôte (aminopeptidase N) et de l'induction de la fusion de l'enveloppe virale avec la membrane cellulaire.

Le FIPV, très proche morphologiquement et antigéniquement du FECV, se distingue de ce dernier en ce qu'il a acquis la capacité à se répliquer dans les macrophages [Pedersen, 1995]. Dans la plupart des cas, les chats ayant développé des anticorps contre FCoV, développent une maladie plus grave et plus rapide, après l'épreuve virulente que les chats séronégatifs (absence d'anticorps anti-FCoV). Ce phénomène de facilitation est dû à la formation d'anticorps facilitant l'infection virale, dirigés contre la protéine S [Pedersen, 1980 ; Olsen et al., 1992] et dont l'action a un effet délétère et contraire à celle des anticorps protecteurs, en formant des complexes immuns qui sont plus infectieux, particulièrement pour les macrophages ; ce phénomène, également dénommé ADE, pour "*antibody-dependant enhancement*", explique probablement, au moins en partie, la faible efficacité des vaccins comprenant la protéine S de coronavirus.

Dans un tel contexte, le développement d'un vaccin sûr et efficace contre les coronavirus félins est donc très problématique.

Or, la péritonite infectieuse féline (PIF) pose un problème important de santé vétérinaire ; en effet, les jeunes chats sont particulièrement sensibles à la PIF : 54 % de tous les cas de PIF concernent des chats ayant moins de 12 mois et 70 % concernent des chats de moins de 4 ans. Parmi ces chats infectés, les infections dues à des souches de coronavirus de type I semblent prédominantes, alors que les infections de type il comptent respectivement pour 5 % et pour 20 à 30 % des cas de FIP aux États-Unis et au Japon [Pedersen et al., 1983; Hohdatsu et al., 1992].

Les deux sérotypes sont caractérisés sur la base de la neutralisation *in vitro.* Les sérums des chats atteints de FCoVs de type I neutralisent d'autres FcoVs de type I, mais pas les FCoVs de type II et vice versa. On pense que les coronavirus félins de type II ont pour origine des évènements de recombinaison d'ARN pendant lesquels le gène de la protéine S du coronavirus canin a été incorporé au génome des FcoVs de type I [Herrewegh et al., 1998].

Selon les symptômes observés, deux formes de la maladie ont été décrites :
- une forme effusive ou humide, dans laquelle on observe une accumulation de liquide d'ascite, provoquée par une réponse inflammatoire intense et l'activation de la cascade du complément (augmentant la perméabilité vasculaire) [Pedersen, 1976 ; Pedersen, 1980] et
- une forme non-effusive ou sèche, qui se caractérise par peu ou pas d'accumulation de liquide d'ascite, mais dans laquelle on observe, généralement, des dépôts fibrineux granulaires sur différents organes (foie, rate, intestin, poumons). Les résultats d'analyses biochimiques et hématologiques montrent le plus souvent : une anémie, une neutrophilie, une lymphopénie, une augmentation des protéines totales du sérum et une hyperglobulinémie s'accompagnant d'une diminution du taux d'albumine.

En l'absence de traitements efficaces, différents vaccins anti-FIPV ont été proposés, qui mettent en oeuvre des stratégies vaccinales distinctes, eu égard au nombre et au type des antigènes mis en oeuvre et/ou au mode et à la forme d'expression et de présentation des antigènes.

On peut citer par exemple, les vaccins incorporant des virus inactivés entiers [Pedersen, 1983], des virus vivants atténués ou des virus hétérologues vivants (CCV, HCV-229E, TGEV), mais aussi des vaccins vectorisés basés sur les poxvirus, le virus de l'herpès félin (FHV) ou l'adénovirus, qui expriment la protéine S, M ou N ou des fragments simples de ces protéines, en combinaison ou en fusion avec d'autres protéines porteuses [Vennema et al., 1991 ; EP 652 287 ; WO 97/20054 ; WO 97/20059 ; Woobs, R.D. et al., 1979 ; Stoddart, C.A. et al., 1988 ; Barlough, J.E. et al., 1985].

Toutefois, de tels vaccins, lorsqu'ils mettent en oeuvre la protéine S, peuvent induire des phénomènes de facilitation par production d'anticorps facilitants, incompatibles avec l'établissement d'une protection efficace.

D'autres voies de recherche de vaccins ont donc été proposées, pour éliminer les anticorps facilitants et ainsi essayer d'induire une immunité humorale efficace.

Ce sont, pour la majorité, des vaccins recombinants sous-unitaires comportant, séparément ou en combinaison, au moins une protéine S, M ou N normale, recombinante ou modifiée ou au moins un peptide inclus dans l'une de ces protéines [WO 97/20054 ; WO 92/08487]. De manière plus précise :
* l'antigène peut être constitué par une protéine S modifiée au niveau des sites ou des épitopes, spécifiquement associés à l'ADE, en partie N-terminale de la protéine S, c'est-à-dire, de manière plus spécifique : le site A1 (aa 562-598), le site A2 (aa 637-662) et éventuellement le site D [Corapi, W.V. et al., 1995 ; WO 95/07987 ; WO 96/06934 ; WO 95/07987 ; WO 93/23421] ; toutefois, cette stratégie a notamment l'inconvénient de reposer sur des mutations dont il est connu qu'elles risquent d'entraîner une perte de l'antigénicité de l'ensemble de la protéine S ;
* l'antigène peut être constitué par une région hautement conservée ou UCD (pour *Universal Conserved Domain*), constituée par la partie C-terminale de 124 acides aminés (résidus de 1077 à 1276) de la protéine S [WO 92/08487 ; WO 93/23421]. La détermination d'une séquence universelle entre différents coronavirus montre effectivement le rôle important de cette dernière dans la structure de la protéine et de là, dans la structure du virus et/ou dans sa réplication. Toutefois, cela ne permet aucunement de prédire son activité immunoprotectrice ; toutefois, ce fragment peut induire la production d'anticorps facilitants.

Le document WO9323423 décrit des fragments d'une protéine S du coronavirus de chien (CCV) et leur utilisation pour la préparation d'un vaccin.

Gonon V et al (1999) décrit la caractérisation d'anticorps anti glyproteine 5 associés à la régression de l'infection par le VPIF.

Aussi, ces différentes stratégies peuvent induire une perte d'antigénicité.

Bien que les raisons exactes pour lesquelles certains des antigènes, apparemment prometteurs, donnent des efficacités relativement faibles, restent à déterminer, elles sont susceptibles d'être multifactorielles. Parmi les facteurs les plus critiques, on trouve le type de l'antigène(s) ou de l'épitope(s). Si l'on considère un épitope approprié, l'immunité cellulaire est présumée être la clé effectrice de la protection (bien qu'il y ait quelques études qui démontrent que des réponses neutralisantes d'anticorps pourraient être la clé principale de la protection *in vivo*). D'autre part, l'antigène devrait être suffisamment immunogène et être capable de le rester en présence d'autres antigènes, compétiteurs ou interférents, comme par exemple d'autres protéines du coronavirus qui seraient nécessaires à la protection ou des antigènes provenant d'autres vaccins ou vaccins associés.

Si la force antigénique peut être améliorée et si la concurrence antigénique peut être surmontée par diverses méthodes, compositions ou combinaisons de celles-ci, la nature protectrice d'un antigène ou d'un épitope, qui sont inhérents à la séquence en acides aminés de l'épitope, demeure invariable, d'où la nécessité du choix judicieux du ou des antigène(s).

En conséquence, aucune des pistes de vaccins précédemment proposées ne présente l'ensemble des conditions requises, à savoir :
- l'obtention d'une immunité humorale protectrice *in vivo*
- l'absence de production d'anticorps facilitants et,
- l'absence de phénomènes de facilitation *in vivo*.

La Demanderesse s'est, en conséquence, donnée pour but la production d'un vaccin qui répond mieux aux besoins de la pratique, notamment en ce qu'il n'induit pas la formation d'anticorps délétères, tout en conservant la capacité à produire une réponse immune protectrice.

La Demanderesse a sélectionné des peptides compris dans la protéine S du coronavirus de la PIF, qui, de manière surprenante, permettent effectivement d'induire une immunité protectrice, sans induire de phénomènes de facilitation.

Pour obtenir un tel vaccin, la démarche qui consiste à rechercher les sites épitopiques que l'on peut généralement déduire par des méthodes basées sur de la modélisation par ordinateur ou par extrapolation de l'antigénicité de la protéine S, à partir d'autres espèces de coronavirus ou par un mélange des deux méthodes, permettant la localisation des séquences les « plus probables », ne permet pas de déduire quels sont les peptides qui ne possèdent pas de propriétés délétères.

La Demanderesse a, en conséquence, mis au point un système qui a permis de sélectionner des peptides compris dans la protéine S particulièrement performants.

La sélection de ces peptides est ainsi liée, de manière surprenante, à la résistance et/ou à la sensibilité des chats aux infections à coronavirus d'une part et à la régression ou à la progression de la PIF d'autre part.

En particulier, la Demanderesse a montré, de manière surprenante, que les peptides sélectionnés étaient reconnus spécifiquement par les sérums de chats spontanément régresseurs (SR).

La presente invention est telle que définie par les revendications.

Les données ci-après résument les correspondances entre les différentes séquences, pour plus de clarté :
- La SEQ ID NO :6 comprend 1452 acides aminés et correspond à la séquence complète de la protéine S de coronavirus de la souche FIPV 79-1146 ;
- La SEQ ID NO:5 comprend 365 acides aminés et correspond aux positions 940-1304 de la séquence SEQ ID NO :6 ;
- Le peptide de SEQ ID NO :2 selon l'invention comprend 21 acides aminés et correspond aux positions 1-21 de la SEQ ID NO :5 ou aux positions 940-960 de la SEQ ID NO :6 ;
- Le peptide de SEQ ID NO :3 selon l'invention comprend 59 acides aminés et correspond aux positions 15-73 de la SEQ ID NO :5 ou aux positions 954-1012 de la SEQ ID NO :6;
- Le peptide de SEQ ID NO :4 de 31 acides aminés correspond aux positions 335-365 de la SEQ ID NO:5 ou aux positions 1274-1304 de SEQ ID NO:6.

En référence aux SEQ ID NO:5 et NO:6 le fragment C-terminal contenant le domaine universel (UCD) et le domaine universel UCD de 124 acides aminés, décrits dans la Demande Internationale WO 93/23421 correspondent aux positions suivantes des SEQ ID NO 5 et 6 :

| fragments de wo 93/23421 | Positions dans seq id no :5 | Positions dans seq id no:6 |
|---|---|---|
| Fragment C-terminal de la protéine S (SEQ ID NO:1-12 de 199 ou 200 acides aminés | Positions 138-337 | Positions 1077-1276 |
| Fragment UCD de 124 acides aminés (positions 37-160 des SEQ ID NO :1-12) | Positions 175-298 | Positions 1114-1237 |

Les peptides selon l'invention sont sélectionnés dans le groupe constitué par le peptide SEQ ID NO:2, les peptides contenant de 12 à 20 acides aminés de la séquence SEQ ID NO:2, le peptide SEQ ID NO:3, les peptides contenant de 12 à 20 acides aminés de la séquence SEQ ID NO:3 Excepté le peptide de séquence LEU-GLY- THR- VAL- ASP-GLU-ASP-TYR-LYS-ARG-CYS- THR- GLY-GLY-TYR- ASP, le peptide SEQ ID NO:4 et les peptides contenant de 12 à 20 acides aminés de la séquence SEQ ID NO:4.

Lesdits peptides sont inclus entre les acides aminés 940 et 1304 de la protéine S, en référence à la protéine S de SEQ ID NO:6 de la souche FIPV 79-1146 et sont donc sélectionnés dans le groupe constitué par le peptide 7I correspondant aux positions 940-960 (SEQ ID NO:2) de la protéine S de SEQ ID NO:6, les peptides contenant de 12 à 20 acides aminés et dont la séquence est contenue dans la SEQ ID NO:2, le peptide T12 correspondant aux positions 954-1012 (SEQ ID NO:3) de la protéine S de SEQ ID NO:6, les peptides contenant de 12 à 20 acides aminés et dont la séquence est contenue dans la SEQ ID NO:3, Excepté le peptide de séquence LEU-GLY- THR- VAL- ASP-GLU-ASP-TYR-LYS-ARG-CYS- THR- GLY-GLY-TYR- ASP, le peptide 14I correspondant aux positions 1274-1304 (SEQ ID NO:4) de la protéine S de SEQ ID NO:6 et les peptides contenant de 12 à 20 acides aminés et dont la séquence est contenue dans la SEQ ID NO:4. De manière surprenante, lesdits peptides :
- n'induisent pas de phénomènes de facilitation, et
- sont aptes à induire une immunité contre des infections à coronavirus, et notamment contre la péritonite infectieuse féline chez les chats.

Aucun des peptides selon l'invention, c'est-à-dire présentant les propriétés d'induction d'anticorps neutralisants et d'absence d'induction d'anticorps facilitants n'inclut le fragment correspondant aux positions 175-298 de la SEQ ID NO :5 ou 1114-1237 de la SEQ ID NO :6, qui peut induire la production d'anticorps facilitants.

En outre, les propriétés immunologiques desdits peptides sont corrélées avec les réactions immuno-protectrices dans un groupe de chats montrant une régression spontanée de l'infection à coronavirus.

Parmi lesdits peptides, le peptide T12 (SEQ ID NO:3) est particulièrement préféré.

Les inventeurs descrivent des peptides, tels que définis ci-dessus, modifiés par des mutations artificielles, des délétions, des insertions, des variations ou des combinaisons de ces événements, à condition que les peptides ainsi modifiés n'induisent pas de phénomènes de facilitation.

L'invention inclut également les peptides tels que définis ci-dessus, sous forme de peptides de synthèse, de peptides répétés, de protéines fusionnées à l'extrémité N- ou C- terminale avec une protéine d'un autre agent pathogène félin (tels que FIV, FeLV, FHV, Calicivirus, Parvovirus, Bordetella, Chlamidia) .

La présente invention a également pour objet un vaccin pour protéger les chats contre la péritonite infectieuse féline (FIP), caractérisé en ce qu'il comprend au moins un peptide tel que défini ci-dessus en combinaison avec des substances porteuses et/ou des adjuvants et/ou au moins un véhicule pharmaceutiquement acceptable.

Les adjuvants utilisés sont des adjuvants classiquement utilisés ; avantageusement, ils sont choisis dans le groupe constitué par des émulsions huileuses, de la saponine, des substances minérales, des extraits bactériens, de l'hydroxyde d'alumine et le squalène.

Les substances porteuses sont avantageusement sélectionnées dans le groupe constitué par des liposomes unilamellaires, des liposomes multilamellaires, des micelles de saponine ou des microsphères solides de nature saccharidique ou aurifère.

Selon un mode de réalisation avantageux dudit vaccin, il comprend en outre d'autres peptides ou protéines virales appropriées.

La présente invention a également pour objet des molécules d'acide nucléique codant pour les différents peptides tels que définis ci-dessus.

De manière plus précise, lesdites molécules d'acide nucléique sont notamment sélectionnées dans le groupe constitué par les séquences SEQ ID NO:7-9 ainsi que les séquences nucléotidiques contenant de 36 à 60 nucléotides et dont la séquence est contenue dans la SEQ ID NO:8 et les séquences nucléotidiques contenant de 36 à 60 nucléotides et dont la séquence est contenue dans la SEQ ID NO:9.

La présente invention a également pour objet l'utilisation desdites molécules d'acide nucléique pour la construction de vecteurs recombinants (virus ou plasmides), utiles comme vaccins.

La présente invention a également pour objet des vecteurs recombinants, caractérisés en ce qu'ils comprennent une molécule d'acide nucléique, telle que définie ci-dessus.

De manière avantageuse, lesdits vecteurs sont, de préférence, sélectionnés dans le groupe constitué par des vecteurs viraux, tels que les poxvirus, les adénovirus, les rétrovirus, les herpès virus, des vecteurs bactériens, tels que les mycobactéries, entérobactéries ou les lactobacilles et/ou des plasmides incluant une séquence codant pour au moins l'un des peptides tels que définis ci-dessus.

La présente invention a également pour objet un vaccin pour protéger les chats contre la péritonite infectieuse féline (FIP), caractérisé en ce qu'il comprend au moins une molécule d'acide nucléique telle que définie ci-dessus ou un vecteur recombinant tel que défini ci-dessus.

Les peptides immunogènes de la protéine S, tels que définis ci-dessus ou les vecteurs recombinants exprimant lesdits peptides sont particulièrement bien adaptés à la prophylaxie des maladies provoquées par les coronavirus, incluant en particulier le FIPV.

De manière avantageuse, lesdits peptides peuvent être obtenus par synthèse chimique ou par recombinaison de l'ADN correspondant dans une bactérie, un virus, une lavure ou un hôte eucaryote.

Les vaccins selon l'invention sont avantageusement administrés par voie systémique (intramusculaire, sous-cutanée intra-péritonéale ou intraveineuse) et/ou par voie locale (orale, nasale, autres voies muqueuses) ou par combinaison de ces voies et induisent effectivement une réponse immune protectrice contre le coronavirus félin FIPV

La présente invention a également pour objet un procédé de sélection de peptides immunogènes correspondant à un fragment d'une Protéine S de coronavirus, et n'induisant pas de phénomènes de facilitation, caractérisé en ce qu'il comprend au moins les étapes suivantes :
- la construction d'une banque aléatoire de peptides correspondant à un fragment de protéine S de coronavirus, à partir d'au moins un génome viral de FIPV,
- la mise en contact desdits peptides avec au moins quatre sérums différents de chats spontanément régresseurs (SR), après une infection par le FIPV, à une dilution d'au moins 1/1000^{ème} et
- l'immuno-sélection des peptides interagissant avec lesdits sérums de chats spontanément régresseurs, mais interagissant peu ou même pas du tout avec des sérums de chats présentant des symptômes cliniques (CS) ou des sérums de chats présentant des signes subcliniques de l'infection chronique (CI) avec FIPV.

Un tel procédé permet l'identification des épitopes qui sont spécifiquement corrélés avec des réponses en anticorps protectrices acquises chez les chats spontanément régresseurs (SR) par rapport aux réactions immunes non-protectrices observées dans les groupes de chats présentant des symptômes cliniques (CS) ou des signes subcliniques de l'infection chronique (CI) avec FIPV [Gonon et al., 1999]. Dans un tel procédé :
- l'utilisation de quatre sérums constitue un protocole statistiquement acceptable ;
- la dilution sélectionnée permet d'atteindre à une spécificité que des dilutions inférieures ne permettraient pas d'obtenir (réactions croisées).

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

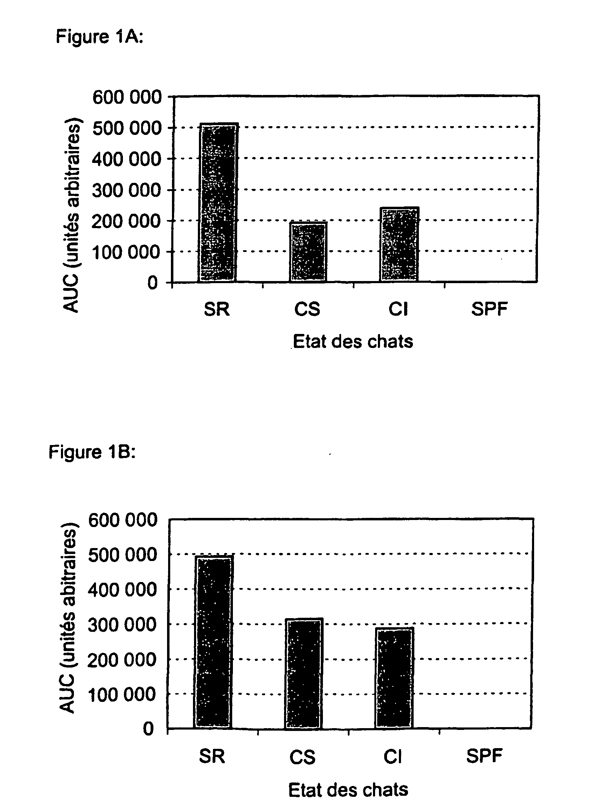

- les Figures 1A et 1B : ces figures illustrent la réactivité des peptides T12 et 7I, avec les différentes catégories de sérums suivantes : SR = chats spontanément régresseurs, CS = chats avec les signes cliniques, CI = chats chroniquement infectés, SPF = chats sans pathogènes spécifiques (contrôle). Des évaluations densitométriques de l'aire sous la courbe (AUC) dans les *Western blots* sont données en unités arbitraires. Les données représentent des valeurs moyennes (n) du nombre d'échantillons de sérums provenant des groupes respectifs de chats ; figure 1A : réponse en anticorps par rapport à T12 ; figure 1B : réponse en anticorps par rapport à 71.

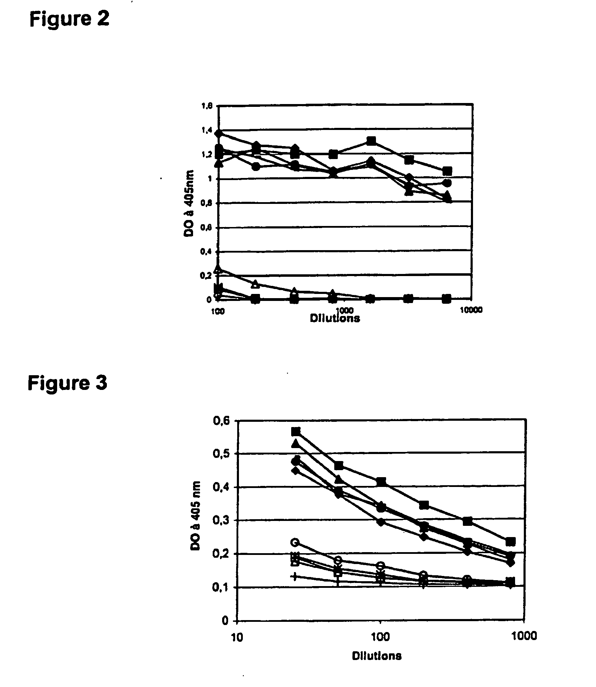

- la Figure 2 illustre les réponses en anticorps contre T12 avant l'épreuve par la souche de FIPV 79-1146. Les cinq chats vaccinés avec le vaccin T12 sont représentés avec les symboles "pleins" suivants ■, ▲, ●, +, ◆. Les cinq chats non vaccinés sont représentés avec les symboles "vides" suivants □, Δ, ○, +, ◊ .

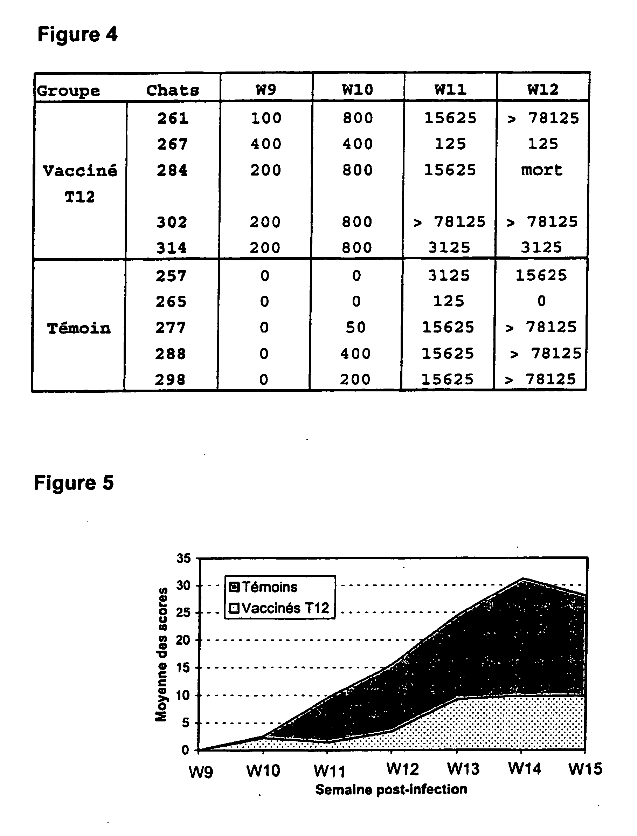

- **la** **Figure 3** illustre les réponses en anticorps contre l'antigène viral (FIPV 79-1146) avant l'épreuve par FIPV79-1146. Les cinq chats vaccinés avec le vaccin T12 sont représentés avec les symboles "pleins" suivants ■, ▲, ●, +, ◆. Les cinq chats non vaccinés sont représentés avec les symboles "vides" suivants □, Δ, ○, +, ◊ .
- **la** **Figure 4** représente les réponses en anticorps dirigés contre l'antigène viral (FIPV 79-1146), après l'épreuve par FIPV souche 79-1146.
- **la** **Figure 5** représente une évaluation dans le temps des signes cliniques, suivant l'épreuve avec FIPV 79-1146. Les chats SPF (5 par groupe) ont été vaccinés en sous-cutanée deux fois à un intervalle de trois semaines avec le vaccin de la sous-unité T12, alors que les chats contrôles (5 par groupe) recevaient le placebo de PBS. Les deux groupes de chats ont été infectés avec FIPV 79-1146 pendant six semaines après la deuxième vaccination et les signes cliniques ont été évalués hebdomadairement.

Il doit être bien entendu, toutefois, que ces exemples sont donnés uniquement à titre d'illustration de l'objet de l'invention, dont ils ne constituent en aucune manière une limitation.

### Exemple 1 : Procédé de sélection des peptides selon l'invention.

### - Caractéristiques des réponses immunologiques chez les chats infectés par un coronavirus

L'expérience a impliqué initialement 150 chats infectés par le coronavirus félin avec un historique connu ou inconnu des signes cliniques, puis a été réduite à 42 chats pendant la période expérimentale qui a duré plus d'une année, conduisant à la collecte de 133 échantillons de sérum appartenant aux catégories CS (42), CI (48), et SR (43). L'analyse par *Western-blot* des protéines virales M, N, et S, a montré que les trois catégories de sérums révélaient un schéma distinct de la réactivité antigénique (Tableau I).

Ces données montrent que la régression est corrélée à la réponse en anticorps anti-S ; en outre, on observe également un rapport anticorps anti-S/anticorps anti-M élevé. En revanche, la progression de la maladie ou de l'infection chronique se caractérise par un rapport anticorps anti-S/anti-M plus faible. Les données indiquent également que les réponses en anticorps contre des protéines M et également N sont généralement dominantes en comparaison avec les réponses contre la protéine S, et ont de faibles valeurs indicatives en ce qui concerne le statut de la maladie. Les données montrent également qu'il doit être techniquement possible de discerner l'épitope(s) protecteur(s) résidant dans la protéine S par *Western-blot* avec des antisérums de SR. Ces épitopes ne sont que très peu ou pas identifiés par des sérums de CS ou de CI.

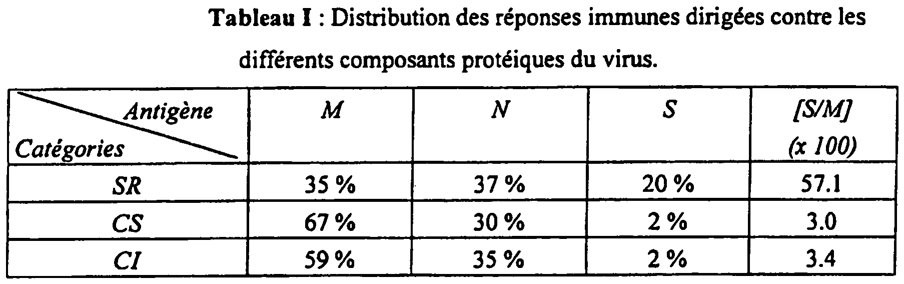

### - Identification de l'épitope(s) impliqué dans les réponses immunes protectrices.

En utilisant les antisérums obtenus à partir des chats SR, les épitopes appropriés ont été sélectionnés à l'aide d'une banque aléatoire de peptides, construite à partir du génome viral de FIPV, souche 79-1146 (transcrit et coupé par une DNase) ; les fragments obtenus sont introduits dans un système d'expression bactérien (NovaTope). Les fragments du gène S aléatoirement obtenus (50-150 paires de bases) ont été isolés par électrophorèse sur gel d'agarose et insérés dans le vecteur plasmidique (pSCREEN-1b) par la méthode de ligation des dA-dT.

Des cellules compétentes de *E*. *coli* (DE3) ont été alors transformées avec le vecteur de recombinaison, ayant pour résultat des transformants de l'ordre de 10⁸ cfu/µg d'ADN.

L'immuno-sélection des colonies, effectuée sur des membranes NC (nitrocellulose) avec 4 sérums différents de SR à une dilution de 1/1000^{ème} a conduit à la sélection d'au moins 20 clones candidats qui ont alors été soumis au séquençage de leur ADN.

Les alignements des séquences d'insertion avec la séquence d'ADN du gène S du FIPV 79-1146 [De Groot, R.J. et al., 1987] ont permis l'identification, entre autres, de trois peptides candidats respectivement appelés 7I (SEQ ID NO :2), T12 (SEQ ID NO :3) et 14I (SEQ ID NO : 4) (Tableau II).

Les deux peptides 71 et T12 partagent une région identique de sept acides aminés. La région C-terminale de la protéine S comportant les trois peptides est fortement conservée entre les sérotypes I et II des coronavirus félins et également parmi les coronavirus canins et porcins [Wesseling, J.G. et al., 1994]. Il est donc très probable que les peptides, s'ils sont immunogènes chez le chat, soient aussi immunogènes dans les pathologies canines, porcines et humaines.

Pour confirmer l'immuno-sélection positive obtenue et afin de caractériser les épitopes protecteurs, les deux peptides appelés 7I et T12 ont été exprimés par *E*. *coli,* sous la forme de protéines de fusion formant des corps d'inclusion, et extraits à partir du lysat de cellules en utilisant l'urée 8 M ; après une électrophorèse sur gel de polyacrylamide, lesdits peptides ont été transférés sur une membrane de nylon et incubés avec les différentes catégories de sérums immuns, tels que défins ci-dessus : SR, CS et CI.

Comme représenté sur la Figure 1 (A et B), les peptides 7I et T12 sont préférentiellement identifiés par des sérums SR, alors qu'ils le sont sensiblement moins par des sérums CS ou CI.

**Tableau II : Identité des séquences des peptides préférentiellement identifiés par les sérums des chats SR (pour spontanément régresseurs).**

| ***SEQ.ID.*** | ***Nom*** | ***Taille (αα)*** | ***Séquence*** | ***Alignement (protéine S*)*** |
|---|---|---|---|---|
| N°2 | 71 | 21 | GGSWLGGLKDILPSH NSKRKY | 940-960 |
| N°3 | T12 | 59 | | 954-1012 |
| N°4 | 14I | 31 | MYQPRVATSSDFVQIEGCDVLFVNATVIDLP | 1274- 1304 |

| | | | | |
|---|---|---|---|---|
| ^{*} Numéroté selon l'ordre de la séquence de 1452 acides aminés de la protéine S (SEQ ID NO:6) (MW = 160 491) de FIPV 79-1146 [De Groot, R.J. et al., 1987]. | | | | |

### EXEMPLE 2 : Préparation de la composition vaccinale.

Une fois purifié ou partiellement purifié, T12 a été utilisé comme vaccin sous-unitaire dans une épreuve virulente réalisée sur des chats. Ce vaccin présente une efficacité protectrice, qui est significative, à partir de la deuxième semaine après l'épreuve virulente, en termes de morbidité, sévérité des symptômes de PIF et particulièrement en terme de mortalité.

Le peptide T12, exprimé dans le système d'induction IPTG de *E*. *coli,* sous la forme d'une protéine recombinante de fusion (MW ∼34 kDa) contenant en C-terminal une étiquette His6, a été extrait à partir du lysat de cellules dans de l'urée 8 M et purifié sur une colonne de résine de Nickel (Qiagen). Le T12 purifié a été alors adsorbé sur hydroxyde d'aluminium (alhydrogel) et formulé dans un tampon phosphate (PBS, pH 7,2) ainsi qu'en présence de saponine (QS21), comme adjuvant.

Une dose vaccinale comporte, pour un volume de 1,0 ml, 100 µg de T12, 10% d'alhydrogel (v/v), et 20 µg de QS21, par chat.

### EXEMPLE 3 : Etude d'une vaccination avec T12, suivie d'une épreuve virulente.

Dans un groupe de cinq chats SPF (HARLAN USA), âgés de 8 à 9 semaines, chaque animal a reçu deux injections par voie sous-cutanée, à un intervalle de trois semaines (W0 et W3). En parallèle, cinq chats témoins (SPF) reçoivent un placebo ne contenant aucune protéine.

Six semaines (W9) après la deuxième vaccination, les deux groupes de chats ont été infectés par voie oro-nasale (moitié par oral et l'autre moitié par nasal) avec 220 TCID₅₀ de la souche FIPV 79-1146. Les animaux ont été suivis toutes les semaines, pendant 8 semaines après l'épreuve, pour la détermination des titres en anticorps (anti-T12 et anti-FIPV) et les signes cliniques (morbidité, aspect des muqueuses, fluide péritonéal, poids, température, hématocrite, leucocytes et mortalité).

Les signes cliniques ont été mesurés selon le schéma décrit dans le Tableau III.

**Tableau III : Schéma d'affectation des points suivant l'apparition et l'intensité des signes cliniques.**

| ***Catégories*** | ***Signes*** | ***Score*** | ***Catégories*** | ***Signes*** | ***Score*** |
|---|---|---|---|---|---|
| Comportement | Normal | 0 | Perte de poids | >20% | 1 |
| | Fatigué | 1 | | >30% | 2 |
| | Très fatigué | 2 | | >50% | 3 |
| | Prostré | 3 | | | |
| Aspect des Muqueuses | Normal | 0 | Température corporelle (°C) | 39.4-39.9 | 1 |
| | Jaune léger | 1 | | 40-40.5 | 2 |
| | Jaune (citron) | 2 | | >40.6 | 3 |
| Liquide péritonéal | Absent | 0 | Hématocrite* | >25% | 0 |
| | Suspicion | 1 | | 25-20% | 1 |
| | Présent | 2 | | <20% | 2 |
| Pertes d'Equilibre** | | 1 | Nombre de leucocytes | Diminution de 50% | 3 |
| Lésions hépatiques*** (*post-mortem*) | | 1 | | | |

| | | | | | |
|---|---|---|---|---|---|
| * Hématocrite normal de chat : de l'ordre de 25 à 45 % ** Pertes d'équilibre : état psychomoteur de l'animal ; une perte d'équilibre indique une atteinte cérébrale. *** Lésions hépatiques : présence de granulomes sur les lobes. | | | | | |

### 1. Réponses humorales observées avant l'épreuve :

* **Réponse anti-T12 :** tous les animaux vaccinés ont développé une réponse élevée en anticorps anti-T12, atteignant le maximum (titre en Elisa > 100 000) à approximativement 3 semaines (W6) après la seconde vaccination et restant relativement stable tout au long de la période d'observation (jusqu'au jour de l'épreuve). En revanche, il n'est pas possible de détecter des anticorps anti-T12 (Figure 2) chez les chats témoins. Par analyse en *Western-Blot,* les sérums des chats vaccinés ont montré qu'ils reconnaissaient la protéine virale S.
* **Réponse anti-virus :** les chats vaccinés avec le peptide T12 ont développé des réponses anti-FIPV avec des titres en anticorps (par ELISA) allant de 100 à 200 à W6, augmentant légèrement à W9, tandis qu'aucun des chats du groupe témoin n'a montré de réponses détectables (Figure 3).

### 2. Réponses humorales développées après l'épreuve virulente

*** Réponse anti-virus :** Quatre des cinq chats vaccinés ont répondu à l'épreuve par une élévation rapide du titre en anticorps anti-FIPV, atteignant 5 000 à 11 000 à 2 semaines (W11) post-infection. Deux des quatre chats répondants ont continué à développer des titres plus élevés à W12 et à W13 (d'environ 80 000) ; tandis qu'un chat est resté stable avec le titre de 5000 et l'autre a succombé à W12. L'animal n'ayant pas répondu, a montré des titres en anticorps qui diminuaient.

Quatre des cinq chats du groupe témoin ont également répondu à l'épreuve avec des réponses fortes en anticorps une à deux semaines plus tard que les chats vaccinés, les titres atteignant des niveaux de 80 000 à W12 et/ou à W13. Le chat non répondant s'est avéré transitoirement séropositif au point W11 avec un titre en anticorps de environ 100 (Figure 4).

### 3. Suivi des signes cliniques pendant l'épreuve :

Les animaux ont été surveillés chaque jour : les signes cliniques et des points ont été attribués pour chaque observation, sur l'échelle de 0 pour des valeurs normales à 3 pour des valeurs anormales et selon la sévérité (voir Tableau III). Comme le montre le Tableau IV, quatre des cinq chats vaccinés (ou 80 %) n'ont pas ou faiblement présenté de signes cliniques, tandis qu'un des chats développait les signes cliniques typiques de la PIF.

Contrairement au groupe vacciné, trois des cinq animaux du groupe témoin ont présenté les signes cliniques caractéristiques de la PIF menant jusqu'à la mort ; un animal a montré des symptômes relativement plus faible, tandis que le dernier (animal transitoirement séropositif à la deuxième semaine) n'a montré aucun signe clinique.

Ces données montrent que le vaccin sous-unitaire T12 est capable de conférer une protection aux chats infectés avec un coronavirus virulent (pas d'apparition d'anticorps facilitants).

Par l'évaluation de la mortalité, le vaccin a montré une efficacité de 80% de survie, comparée à 40% dans le groupe témoin. Parmi les survies, certains animaux vaccinés étaient complètement ou presque totalement exempts de symptômes, tandis qu'un des deux chats témoins survivants a développé des symptômes faibles.

De plus, le suivi au cours du temps des signes cliniques suggère que la protection induite par le vaccin commence à apparaître à partir de la deuxième semaine (W11) post-infection (Figure 5).

**Tableau IV : Scores totaux observés sur les six premières semaines d'infection.**

| ***Numéro Chat*** | ***Températu re*** | ***Perte de poids*** | ***Nombre de leucocyt es*** | ***Hématocrite*** | ***Symptôme (****)*** | ***Score Total(*)*** |
|---|---|---|---|---|---|---|
| **Vaccinés T12** | | | | | | |
| 261 | 0 | 0 | 6 | 1 | 0 | **7** |
| 267 | 0 | 0 | 0 | 0 | 0 | **0** |
| 284 | 0 | 0 | 6 | 2 | 158 | **166*** |
| 302 | 0 | 0 | 6 | 0 | 0 | **6** |
| 314 | 0 | 0 | 0 | 0 | 0 | **0** |

| **Non vaccinés** | | | | | | |
|---|---|---|---|---|---|---|
| 257 | 0 | 0 | 12 | 7 | 186 | **205*** |
| 265 | 0 | 0 | 0 | 0 | 0 | **0** |
| 277 | 1 | 1 | 6 | 3 | 159 | **170*** |
| 288 | 2 | 2 | 6 | 2 | 132 | **144*** |
| 298 | 1 | 0 | 9 | 0 | 29 | **39** |

| | | | | | | |
|---|---|---|---|---|---|---|
| ^{*} Chats morts avec des symptômes caractéristiques de la PIF à la 5^{ème} ou 6^{ème} semaine post-infection. ** Addition des scores observés pour le comportement, l'aspect des muqueuses, la présence de liquide péritonéal, la perte d'équilibre et les lésions hépatiques. | | | | | | |

De manière générale, les valeurs des scores sont comprises entre 0 et 3 ; pour certains critères, on note simplement la présence (1) ou l'absence (0) du critère. Dans le tableau IV, les scores totaux sont une addition des scores qui ont été relevés chaque semaine, après l'épreuve. Un total de 7 correspond à la somme : W9=0, W10=1, W11=2, W12=2, W13=2, W14=0, (car le chat est mort).

### Bibliographie

1. Addie, D.D. et al., (1992a) Vet.Rec. 130 :133. *A study of naturally occuring feline coronavirus infections in kittens.*
2. Addie, D.D. et al., (1992b) Vet.Rec. 131 :202. *Feline coronavirus antibodies in cats.*
3. Barlough, J.E. et al., (1985) Can.J.Comp.Med. 49:303-307. *Experimental inoculation of cats with human coronavirus 229E and subsequent challenge with feline infectious peritonitis virus.*
4. Corapi, W.V. et al., (1995) J.Virol. 69(5): 2858-2862. *Localization of antigenic sites of the S glycoprotein of Feline Infectious Peritonitis Virus involved in neutralization and antibody-dependent enhancement.*
5. De Groot, R.J. et al., (1987) J.Gen.Virol. 68:2639-2646. *cDNA cloning and sequence analysis of the gene encoding the peplomer protein of feline infectious peritonitis virus.*
6. Gonon, V. et al. (1999) J.Gen.Virol. 80 : 2315-2317. *Clearance of infection in cats naturally infected with feline coronaviruses is associated with an anti-S glycoprotein antibody response.*
7. Herrewegh, A.A.P.M. et al., (1998) J.Virol. 72 : 4508-4514. *Feline coronavirus type II strains 79-1683 and 79-1146 originate from a double recombinaison between feline coronavirus type I and canine coronavirus.*
8. Hohdatsu, T. et al., (1992) J.Vet.Med.Sci. 54 :557. *The prevalence of types I and II feline coronavirus infections in cats.*
9. Motokawa, K. et al., (1996). Microbiol. Immunol. 40:425-433. *Comparison of the amino acid sequence and phylogenetic analysis of the peplomer, integral membrane and nucleocapsid proteins of feline, canine and porcine coronaviruses.*
10. Olsen, C.W. et al., (1992) J.Virol. 66:956 - 965. *Monoclonal antibodies to the spike protein of feline infectious peritonitis virus mediate antibody-dependent enhancement of infection of feline macrophages.*
11. Pedersen, N.C. (1976) Feline Pract. 6 : 42. *Feline infectious peritonitis : Something old, something new.*
12. Pedersen, N.C. (1995) Feline Pract. 23:7-20. *An overview of feline enteric coronavirus and infectious peritonitis virus infections.*
13. Pedersen, N.C. (1988) In : N.C.Pedersen (Editor), Feline infectious diseases, American Veterinary Publications, Santa Barbara, CA, pp.45-59. *Feline infectious peritonitis.*
14. Pedersen, N.C. et al. (1983) Am.J.Vet.Res. 44 :229-234. *Attempted immunization of cats against feline infectious peritonitis, using avirulent live virus or sublethal amounts of virulent virus.*
15. Pedersen, N.C. et al. (1980) Am.J.Vet.Res. 41:868-876. *Immunologic phenomena in the effusive form of feline infectious peritonitis.*
16. Pedersen, N.C. et al. (1985) Comp.Cont.Edu. 7:1001-1011. *Experimental studies with three new strains of feline infectious peritonitis virus : FIPV-UCD2, FIPV-UCD3, and FIPV-UCD4.*
17. Pedersen, N.C. et al., (1983) In : Molecular Biology and Pathogenisis of Coronaviruses, Plenum Press, New York, pp.365-380. *Pathogenic differences between various feline coronavirus isolates.*
18. Poland, A.M. et al., (1996) J.Clin.Microbiol. 34 : 3180-3184. *Two related strains of feline infectious peroitonitis virus isolated from immunocompromised cats infected with a feline enteric coronavirus.*
19. Stoddart, C.A. et al. (1988) Res.Vet.Sci. 45:383-388. *Attempted immunization of cats against feline infectious peritonitis using canine coronavirus.*
20. Vennema, H. et al., (1991) Virology 181: 327-335. *Primary structure of the membrane and nucleocapsid protein genes offeline infectious peritonitis virus and immunogenicity of recombinant vaccinia viruses in kittens.*
21. Vennema, H. et al. (1995) Feline Pract. 23 : 40-44. *A comparison of the genomes of FeCVs and FIPVs and what they tell us about the relationships between feline coronaviruses and their evolution.*
22. Wesseling, J.G. et al., (1994) J.Gen.Virol.75: 1789-1794. *Nucleotide sequence and expression of the spicke (S) gene of canine coronavirus and comparaion with the S proteins of feline and porcine coronaviruses.*
23. Woods, R.D. and Pedersen, N.C. (1979) Vet.Microbiol. 4:11-16. *Cross protectionstudies between feline infectious peritonitis and porcine transmissible gastroenteritis.*

## Revendications

1. Peptide constitué par un fragment d'une protéine S du coronavirus de la péritonite infectieuse féline, **caractérisé en ce qu'**il est sélectionné dans le groupe constitué par le peptide SEQ ID NO:2, les peptides de 12 à 20 acides aminés dont la séquence est contenue dans la SEQ ID NO:2, le peptide SEQ ID NO:3, les peptides de 12 à 20 acides aminés dont la séquence est contenue dans la SEQ ID NO:3 excepté le peptide de séquence Leu-Gly-Thr-Val-Asp-Glu-Asp-Tyr-Lys-Arg-Cys-Thr-Gly-Gly-Tyr-Asp, le peptide SEQ ID NO:4 et les peptides de 12 à 20 acides aminés dont la séquence est contenue dans la SEQ ID NO:4.

2. Peptide selon la revendication 1, **caractérisé en ce qu'**il se présente sous forme de peptide de synthèse, de peptides répétés ou de protéine fusionnée à l'extrémité N- ou C-terminale avec une protéine d'un autre agent pathogène félin.

3. Utilisation d'au moins un peptide sélectionné dans le groupe constitué par :
a) un peptide constitué par un fragment d'une protéine S du coronavirus de la péritonite infectieuse féline, **caractérisé en ce qu'**il est sélectionné dans le groupe constitué par le peptide SEQ ID NO:2, les peptides de 12 à 20 acides aminés dont la séquence est contenue dans la SEQ ID NO:2, le peptide SEQ ID NO:3, les peptides de 12 à 20 acides aminés dont la séquence est contenue dans la SEQ ID NO:3, le peptide SEQ ID NO:4 et les peptides de 12 à 20 acides aminés dont la séquence est contenue dans la SEQ ID NO:4, et
b) un peptide tel que défini au paragraphe a), se présentant sous forme de peptide de synthèse, de peptides répétés ou de protéine fusionnée à l'extrémité N- ou C-terminale avec une protéine d'un autre agent pathogène félin,
pour la préparation d'un vaccin pour protéger les chats contre la péritonite infectieuse féline.

4. Vaccin pour protéger les chats contre la péritonite infectieuse féline (FIP), **caractérisé en ce qu'**il comprend au moins un peptide selon l'une quelconque des revendications 1 et 2, en combinaison avec des substances porteuses et/ou des adjuvants et/ou au moins un véhicule pharmaceutiquement acceptable.

5. Vaccin selon la revendication 4, **caractérisé en ce que** lesdits adjuvants sont sélectionnés dans le groupe constitué par des émulsions huileuses, de la saponine, des substances minérales, des extraits bactériens, de l'hydroxyde d'alumine et le squalène.

6. Vaccin selon la revendication 4 ou la revendication 5, **caractérisé en ce que** les substances porteuses sont sélectionnées dans le groupe constitué par des liposomes unilamellaires, des liposomes multilamellaires, des micelles de saponine ou des microsphères solides de nature saccharidique ou aurifère.

7. Vaccin selon l'une quelconque des revendications 4 à 6, **caractérisé en ce qu'**il comprend en outre d'autres peptides ou protéines virales.

8. Molécule d'acide nucléique codant pour un peptide selon l'une quelconque des revendications 1 et 2.

9. Molécule d'acide nucléique selon la revendication 8, **caractérisée en ce qu'**elle est sélectionnée dans le groupe constitué par les séquences SEQ ID NO:7-9 ainsi que les séquences nucléotidiques de 36 à 60 nucléotides dont la séquence est contenue dans la SEQ ID NO :8 et les séquences nucléotidiques de 36 à 60 nucléotides dont la séquence est contenue dans la SEQ ID NO :9.

10. Utilisation d'au moins une molécule d'acide nucléique selon les revendications 8 ou 9 pour la construction de vecteurs recombinants, utiles comme vaccins.

11. Utilisation d'au moins une molécule d'acide nucléique sélectionné dans le groupe constitué par :
- une molécule d'acide nucléique codant pour un peptide tel que défini à la revendication 3, et
- une molécule d'acide nucléique selon la revendication 9,
pour la préparation d'un vaccin pour protéger les chats contre la péritonite infectieuse féline.

12. Vecteurs recombinants, **caractérisés en ce qu'**ils comprennent une molécule d'acide nucléique selon les revendications 8 ou 9.

13. Vecteurs selon la revendication 12, **caractérisés en ce qu'**ils sont sélectionnés dans le groupe constitué par des vecteurs viraux, tels que les poxvirus, les adénovirus, les rétrovirus, les herpès virus, des vecteurs bactériens, tels que les mycobactéries, entérobactéries ou les lactobacilles et/ou des plasmides incluant une séquence codant pour au moins l'un des peptides selon l'une quelconque des revendications 1 et 2.

14. Vaccin pour protéger les chats contre la péritonite infectieuse féline (FIP), **caractérisé en ce qu'**il comprend au moins une molécule d'acide nucléique selon la revendication 8 ou la revendication 9 ou un vecteur recombinant selon la revendication 12 ou la revendication 13.

15. Procédé de sélection de peptides immunogènes correspondant à un fragment d'une protéine S de coronavirus, et n'induisant pas de phénomènes de facilitation, **caractérisé en ce qu'**il comprend au moins les étapes suivantes :
- la construction d'une banque aléatoire de peptides correspondant à un fragment de protéine S de coronavirus, à partir d'au moins un génome viral de virus de la péritonite infectieuse féline (FIPV),
- la mise en contact desdits peptides avec au moins quatre sérums différents de chats spontanément régresseurs (SR), après une infection par le FIPV, à une dilution d'au moins 1/1000^{ème} et
- l'immuno-sélection des peptides interagissant avec lesdits sérums de chats spontanément régresseurs, mais interagissant peu ou même pas du tout avec des sérums de chats présentant des symptômes cliniques (CS) ou des sérums de chats présentant des signes subcliniques de l'infection chronique (CI) avec FIPV.

## Claims

1. Peptide consisting of a fragment of an S protein of the feline infectious peritonitis Coronavirus, **characterised in that** it is selected from among the peptide SEQ ID NO. 2, the peptides having 12 to 20 amino acids the sequence of which is contained in SEQ ID NO. 2, the peptide SEQ ID NO. 3, the peptides having 12 to 20 amino acids the sequence of which is contained in SEQ ID NO. 3, with the exception of the peptide of sequence Leu-Gly-Thr-Val-Asp-Glu-Asp-Tyr-Lys-Arg-Cys-Thr-Gly-Gly-Tyr-Asp, the peptide SEQ ID NO. 4 and the peptides having 12 to 20 amino acids the sequence of which is contained in SEQ ID NO. 4.

2. Peptide according to claim 1, **characterised in that** it is in the form of a synthetic peptide, repeat peptides or protein fused at its N- or C-terminal end to a protein of another feline pathogenic agent.

3. Use of at least one peptide selected from among:
a) a peptide consisting of a fragment of an S protein of the feline infectious peritonitis Coronavirus, **characterised in that** it is selected from among the peptide SEQ ID NO. 2, the peptides having 12 to 20 amino acids the sequence of which is contained in SEQ ID NO. 2, the peptide SEQ ID NO. 3, the peptides having 12 to 20 amino acids the sequence of which is contained in SEQ ID NO. 3, the peptide SEQ ID NO. 4 and the peptides having 12 to 20 amino acids the sequence of which is contained in SEQ ID NO. 4, and
b) a peptide as defined in paragraph a), in the form of a synthetic peptide, repeat peptides or protein fused at its N- or C-terminal end to a protein of another feline pathogenic agent,
for the preparation of a vaccine for protecting cats from feline infectious peritonitis.

4. Vaccine for protecting cats from feline infectious peritonitis (FIP), **characterised in that** it comprises at least one peptide according to any one of claims 1 and 2, in combination with carriers and/or adjuvants and/or at least one pharmaceutically acceptable vehicle.

5. Vaccine according to claim 4, **characterised in that** the said adjuvants are selected from among oily emulsions, saponin, mineral substances, bacterial extracts, aluminium hydroxide and squalene.

6. Vaccine according to claim 4 or 5, **characterised in that** the carrier substances are selected from among the unilamellar liposomes, multilamellar liposomes, saponin micelles or solid microspheres of a saccharide or auriferous nature.

7. Vaccine according to any one of claims 4 to 6, **characterised in that** it further comprises other peptides or viral proteins.

8. Nucleic acid molecule coding for a peptide according to any one of claims 1 and 2.

9. Nucleic acid molecule according to claim 8, **characterised in that** it is selected from among the sequences SEQ ID NOS. 7-9 and the nucleotide sequences having 36 to 60 nucleotides the sequence of which is contained in SEQ ID NO. 8 and the nucleotide sequences having 36 to 60 nucleotides the sequence of which is contained in SEQ ID NO. 9.

10. Use of at least one nucleic acid molecule according to claims 8 or 9 for the construction of recombinant vectors that can be used as vaccines.

11. Use of at least one nucleic acid molecule selected from among :
- a nucleic acid molecule coding for a peptide as defined in claim 3, and
- a nucleic acid molecule according to claim 9,
for preparing a vaccine for protecting cats from feline infections peritonitis.

12. Recombinant vectors, **characterised in that** they comprise a nucleic acid molecule according to claims 8 or 9.

13. Vectors according to claim 12, **characterised in that** they are selected from among the viral vectors such as poxviruses, adenoviruses, retroviruses, herpes viruses, bacterial viruses such as mycobacteria, enterobacteria or lactobacilli, and/or plasmids including a sequence coding for at least one of the peptides according to any one of claims 1 and 2.

14. Vaccine for protecting cats from feline infectious peritonitis (FIP), **characterised in that** it comprises at least one nucleic acid molecule according to claim 8 or claim 9 or a recombinant vector according to claim 12 or claim 13.

15. Process for selecting immunogenic peptides corresponding to a fragment of an S protein of Coronavirus, and not inducing any facilitation phenomena, **characterised in that** it comprises at least the following steps:
- constructing a random bank of peptides corresponding to a fragment of an S protein of Coronavirus, from at least one viral genome of feline infectious peritonitis virus (FIPV),
- contacting the said peptides with at least four different spontaneously regressive (SR) cat serums, after infection by FIPV, at a dilution of at least 1/1000th and
- immunoselecting peptides interacting with said spontaneously regressive cat serums but interacting little or not at all with cat serums showing clinical symptoms (CS) or cat serums showing sub-clinical signs of chronic infection (CI) with FIPV.

## Patentansprüche

1. Peptid, bestehend aus einem Fragment eines Proteins S des Coronavirus der felinen infektiösen Peritonitis, **dadurch gekennzeichnet , dass** es aus der Gruppe, bestehend aus Peptid SEQ ID NO:2, Peptiden mit 12 bis 20 Aminosäuren, deren Sequenz in SEQ ID NO:2 enthalten ist, Peptid SEQ ID NO:3, Peptiden mit 12 bis 20 Aminosäuren, deren Sequenz in SEQ ID NO:3 enthalten ist, ausgenommen das Peptid mit der Sequenz Leu-Gly-Thr-Val-Asp-Glu-Asp-Tyr-Lys-Arg-Cys-Thr-Gly-Gly-Tyr-Asp, Peptid SEQ ID NO:4 und Peptiden mit 12 bis 20 Aminosäuren, deren Sequenz in SEQ ID NO:4 enthalten ist, ausgewählt ist.

2. Peptid gemäß Anspruch 1, **dadurch gekennzeichnet , dass** es in Form von synthetischem Peptid, Wiederholungspeptiden oder Protein, das am N- oder C-terminalen Ende mit einem Protein eines anderen felinen pathogenen Agens fusioniert ist, vorliegt.

3. Verwendung wenigstens eines Peptids, ausgewählt aus der Gruppe, bestehend aus:
a) einem Peptid, bestehend aus einem Fragment eines Proteins S des Coronavirus der felinen infektiösen Peritonitis, **dadurch gekennzeichnet , dass** es aus der Gruppe, bestehend aus Peptid SEQ ID NO:2, Peptiden mit 12 bis 20 Aminosäuren, deren Sequenz in SEQ ID NO:2 enthalten ist, Peptid SEQ ID NO:3, Peptiden mit 12 bis 20 Aminosäuren, deren Sequenz in SEQ ID NO:3 enthalten ist, Peptid SEQ ID NO:4 und Peptiden mit 12 bis 20 Aminosäuren, deren Sequenz in SEQ ID NO:4 enthalten ist, ausgewählt ist, und
b) einem Peptid, wie es in Abschnitt a) definiert ist, das in Form von synthetischem Peptid, Wiederholungspeptiden oder Protein, das am N- oder C-terminalen Ende mit einem Protein eines anderen felinen pathogenen Agens fusioniert ist, vorliegt,
zur Herstellung eines Vakzins, um Katzen gegen feline infektiöse Peritonitis zu schützen.

4. Vakzin, um Katzen gegen feline infektiöse Peritonitis (FIP) zu schützen, **dadurch gekennzeichnet, dass** es wenigstens ein Peptid gemäß einem der Ansprüche 1 und 2 in Kombination mit Trägersubstanzen und/oder Adjuvantien und/oder wenigstens einem pharmazeutisch verträglichen Vehikel umfasst.

5. Vakzin gemäß Anspruch 4, **dadurch gekennzeichnet , dass** die Adjuvantien aus der Gruppe, bestehend aus öligen Emulsionen, Saponin, mineralischen Substanzen, Bakterienextrakten, Aluminiumhydroxid und Squalen, ausgewählt sind.

6. Vakzin gemäß Anspruch 4 oder Anspruch 5, dadurch g e - **kennzeichnet,** dass die Trägersubstanzen aus der Gruppe, bestehend aus unilamellaren Liposomen, multilamellaren Liposomen, Saponinmizellen oder festen Mikrokügelchen saccharidischer oder goldhaltiger Natur, ausgewählt sind.

7. Vakzin gemäß einem der Ansprüche 4 bis 6, dadurch g e - **kennzeichnet,** dass es außerdem andere virale Peptide oder Proteine umfasst.

8. Nukleinsäuremolekül, das für ein Peptid gemäß einem der Ansprüche 1 und 2 codiert.

9. Nukleinsäuremolekül gemäß Anspruch 8, dadurch g e - **kennzeichnet,** dass es aus der Gruppe, bestehend aus Sequenzen SEQ ID NO:7-9 sowie Nukleotidsequenzen mit 36 bis 60 Nukleotiden, deren Sequenz in SEQ ID NO:8 enthalten ist, und Nukleotidsequenzen mit 36 bis 60 Nukleotiden, deren Sequenz in SEQ ID NO:9 enthalten ist, ausgewählt ist.

10. Verwendung wenigstens eines Nukleinsäuremoleküls gemäß den Ansprüchen 8 oder 9 für die Konstruktion von rekombinanten Vektoren, die als Vakzine verwendbar sind.

11. Verwendung wenigstens eines Nukleinsäuremoleküls, ausgewählt aus der Gruppe, bestehend aus:
- einem Nukleinsäuremolekül, das für ein Peptid, wie es in Anspruch 3 definiert ist, codiert, und
- einem Nukleinsäuremolekül gemäß Anspruch 9,
für die Herstellung eines Vakzins, um Katzen gegen feline infektiöse Peritonitis zu schützen.

12. Rekombinante Vektoren, **dadurch gekennzeichnet , dass** sie ein Nukleinsäuremolekül gemäß einem der Ansprüche 8 oder 9 umfassen.

13. Vektoren gemäß Anspruch 12, dadurch **gekennzeich** - **net**, dass sie aus der Gruppe ausgewählt sind, bestehend aus Poxviren, Adenoviren, Retroviren, Herpesviren, bakteriellen Vektoren wie Mycobakterien, Enterobakterien oder Lactobacilli und/oder Plasmiden, die eine Sequenz umfassen, die für wenigstens eines der Peptide gemäß einem der Ansprüche 1 und 2 codiert.

14. Vakzin, um Katzen gegen feline infektiöse Peritonitis (FIP) zu schützen, **dadurch gekennzeichnet , dass** es wenigstens ein Nukleinsäuremolekül gemäß Anspruch 8 oder Anspruch 9 oder einen rekombinanten Vektor gemäß Anspruch 12 oder Anspruch 13 umfasst.

15. Verfahren zur Selektion von immunogenen Peptiden, die einem Fragment eines Proteins S von Coronavirus entsprechen und die keine Fazilationsphänomene induzieren, **dadurch gekennzeichnet, dass** es wenigstens die folgenden Stufen umfasst:
- Konstruktion einer zufälligen Bank von Peptiden, die einem Fragment des Proteins S von Coronavirus entsprechen, ausgehend von wenigstens einem viralen Genom des Virus der felinen infektiösen Peritonitis (FIPV),
- Inkontaktbringen der Peptide mit wenigstens vier verschiedenen Seren von spontan regressiven Katzen (SR), nach einer Infektion durch FIPV, mit einer Verdünnung von wenigstens 1/1000 und
- Immunoselektion der Peptide, die den Seren von spontan regressiven wechselwirken, die aber wenig oder sogar überhaupt nicht mit Seren von Katzen, die klinische Symptome (CS) zeigen, oder Seren von Katzen, die subklinische Symptome der chronischen Infektion (CI) mit FIPV zeigen, wechselwirken.


### LISTE DE SEQUENCES

<110> VIRBAC
   INSTITUT NATIONAL DE LA RECHERCHE AGRONOMIQUE-INRA
   ECOLE NATIONALE VETERINAIRE DE MAISONS-ALFORT-ENVA
   AUBERT André
   DUQUESNE Véronique
   ELOIT Marc
   GONON Valérie
<120> VACCIN ANTI-CORONAVIRUS
<130> 004cas70FR
<140>
   <141>
<160> 10
<170> PatentIn Ver. 2.1
<210> 1
   <211> 4500
   <212> ADN
   <213> coronavirus de la péritonite infectieuse féline (protéine S)
<220>
   <221> CDS
   <222> (70)..(4428)
<400> 1
   gaagggtaag ttactcatta gaaataatgg caagctacta aactttggta atcatttagt 60
   taatgtgcc atg att gtg ctc gta act tgc ctc ttg ttg tta tgt tca tac 111
             Met Ile Val Leu Val Thr Cys Leu Leu Leu Leu Cys Ser Tyr
               1               5                  10
   cac aca gtt ttg agt aca aca aat aat gaa tgc ata caa gtt aac gta   159
   His Thr Val Leu Ser Thr Thr Asn Asn Glu Cys Ile Gln Val Asn Val
    15                  20                  25                  30
   aca caa ttg gct ggc aat gaa aac ctt atc aga gat ttt ctg ttt agt   207
   Thr Gln Leu Ala Gly Asn Glu Asn Leu Ile Arg Asp Phe Leu Phe Ser
                    35                  40                  45
   aac ttt aaa gaa gaa gga agt gta gtt gtt ggt ggt tat tac cct aca   255
   Asn Phe Lys Glu Glu Gly Ser Val Val Val Gly Gly Tyr Tyr Pro Thr
                50                  55                  60
   gag gtg tgg tac aac tgc tct aga aca gct cga act act gcc ttt cag   303
   Glu Val Trp Tyr Asn Cys Ser Arg Thr Ala Arg Thr Thr Ala Phe Gln
            65                  70                  75
   tat ttt aat aat ata cat gcc ttt tat ttt gtt atg gaa gcc atg gaa   351
   Tyr Phe Asn Asn Ile His Ala Phe Tyr Phe Val Met Glu Ala Met Glu
        80                  85                  90
   aat agc act ggt aat gca cgt ggt aaa cca tta tta ttt cat gtg cat    399
   Asn Ser Thr Gly Asn Ala Arg Gly Lys Pro Leu Leu Phe His Val His
    95                 100                 105                 110
   ggt gag cct gtt agt gtt att ata tcg gct tat agg gat gat gtg caa    447
   Gly Glu Pro Val Ser Val Ile Ile Ser Ala Tyr Arg Asp Asp Val Gln
                   115                 120                 125
   caa agg ccc ctt tta aaa cat ggg tta gtg tgc ata act aaa aat cgc    495
   Gln Arg Pro Leu Leu Lys His Gly Leu Val Cys Ile Thr Lys Asn Arg
               130                 135                 140
   cat att aac tat gaa caa ttc acc tcc aac cag tgg aat tcc aca tgt    543
   His Ile Asn Tyr Glu Gln Phe Thr Ser Asn Gln Trp Asn Ser Thr Cys
           145                 150                 155
   acg ggt gct gac aga aaa att cct ttc tct gtc ata ccc acg gac aat    591
   Thr Gly Ala Asp Arg Lys Ile Pro Phe Ser Val Ile Pro Thr Asp Asn
       160                 165                 170
   gga aca aaa atc tat ggt ctt gag tgg aat gat gac ttt gtt aca gct    639
   Gly Thr Lys Ile Tyr Gly Leu Glu Trp Asn Asp Asp Phe Val Thr Ala
   175                 180                 185                 190
   tat att agt ggt cgt tct tat cac ttg aac atc aat act aat tgg ttt    687
   Tyr Ile Ser Gly Arg Ser Tyr His Leu Asn Ile Asn Thr Asn Trp Phe
                   195                 200                 205
   aac aat gtc aca ctt ttg tat tca cgc tca agc act gct acc tgg gaa    735
   Asn Asn Val Thr Leu Leu Tyr Ser Arg Ser Ser Thr Ala Thr Trp Glu
               210                 215                 220
   tac agt gct gca tat gct tac caa ggt gtt tct aac ttc act tat tac    783
   Tyr Ser Ala Ala Tyr Ala Tyr Gln Gly Val Ser Asn Phe Thr Tyr Tyr
           225                 230                 235
   aag tta aat aac acc aat ggt cta aaa acc tat gaa tta tgt gaa gat    831
   Lys Leu Asn Asn Thr Asn Gly Leu Lys Thr Tyr Glu Leu Cys Glu Asp
       240                 245                 250
   tat gaa cat tgc act ggc tat gct acc aat gta ttt gct ccg aca tca    879
   Tyr Glu His Cys Thr Gly Tyr Ala Thr Asn Val Phe Ala Pro Thr Ser
   255                 260                 265                 270
   ggt ggt tac ata cct gat gga ttt agt ttt aac aat tgg ttc ttg ctt    927
   Gly Gly Tyr Ile Pro Asp Gly Phe Ser Phe Asn Asn Trp Phe Leu Leu
                   275                 280                 285
   aca aat agt tcc act ttt gtt agt ggc agg ttt gta aca aat caa cca    975
   Thr Asn Ser Ser Thr Phe Val Ser Gly Arg Phe Val Thr Asn Gln Pro
               290                 295                 300
   tta ttg att aat tgc ttg tgg cca gtg ccc agt ttt ggt gta gca gca    1023
   Leu Leu Ile Asn Cys Leu Trp Pro Val Pro Ser Phe Gly Val Ala Ala
           305                 310                 315
   caa gaa ttt tgt ttt gaa ggt gca cag ttt agc caa tgt aat ggt gtg    1071
   Gln Glu Phe Cys Phe Glu Gly Ala Gln Phe Ser Gln Cys Asn Gly Val
       320                 325                 330
   tct tta aat aac aca gtg gat gtt att aga ttc aac ctt aat ttc act    1119
   Ser Leu Asn Asn Thr Val Asp Val Ile Arg Phe Asn Leu Asn Phe Thr
   335                 340                 345                 350
   gca gat gta caa tct ggt atg ggt gct aca gta ttt tca ctg aat aca    1167
   Ala Asp Val Gln Ser Gly Met Gly Ala Thr Val Phe Ser Leu Asn Thr
                   355                 360                 365
   aca ggt ggt gtc att ctt gaa att tca tgt tat agt gac aca gtg agt    1215
   Thr Gly Gly Val Ile Leu Glu Ile Ser Cys Tyr Ser Asp Thr Val Ser
               370                 375                 380
   gag tct agt tct tac agt tat ggt gaa atc ccg ttc ggc ata act gac    1263
   Glu Ser Ser Ser Tyr Ser Tyr Gly Glu Ile Pro Phe Gly Ile Thr Asp
           385                 390                 395
   gga cca cga tac tgt tat gta ctt tac aat ggc aca gct ctt aaa tat    1311
   Gly Pro Arg Tyr Cys Tyr Val Leu Tyr Asn Gly Thr Ala Leu Lys Tyr
       400                 405                 410
   tta gga aca tta cca ccc agt gta aag gaa att gct att agt aag tgg    1359
   Leu Gly Thr Leu Pro Pro Ser Val Lys Glu Ile Ala Ile Ser Lys Trp
   415                 420                 425                 430
   ggc cat ttt tat att aat ggt tac aat ttc ttt agc aca ttt cct att    1407
   Gly His Phe Tyr Ile Asn Gly Tyr Asn Phe Phe Ser Thr Phe Pro Ile
                   435                 440                 445
   ggt tgt ata tct ttt aat tta acc act ggt gtt agt gga gct ttt tgg    1455
   Gly Cys Ile Ser Phe Asn Leu Thr Thr Gly Val Ser Gly Ala Phe Trp
               450                 455                 460
   aca att gct tac aca tcg tat act gaa gca tta gta caa gtt gaa aac    1503
   Thr Ile Ala Tyr Thr Ser Tyr Thr Glu Ala Leu Val Gln Val Glu Asn
           465                 470                 475
   aca gct att aaa aat gtg acg tat tgt aac agt cac att aat aac att    1551
   Thr Ala Ile Lys Asn Val Thr Tyr Cys Asn Ser His Ile Asn Asn Ile
       480                 485                 490
   aaa tgt tct caa ctt act gct aat ttg aat aat gga ttt tat cct gtt    1599
   Lys Cys Ser Gln Leu Thr Ala Asn Leu Asn Asn Gly Phe Tyr Pro Val
   495                 500                 505                 510
   gct tca agt gaa gta ggt ttc gtt aat aag agt gtt gtg tta tta cct    1647
   Ala Ser Ser Glu Val Gly Phe Val Asn Lys Ser Val Val Leu Leu Pro
                   515                 520                 525
   agc ttt ttc aca tac acc gct gtc aat ata acc att gat ctt ggt atg    1695
   Ser Phe Phe Thr Tyr Thr Ala Val Asn Ile Thr Ile Asp Leu Gly Met
               530                 535                 540
   aag ctt agt ggt tat ggt caa ccc ata gcc tcg aca cta agt aac atc    1743
   Lys Leu Ser Gly Tyr Gly Gln Pro Ile Ala Ser Thr Leu Ser Asn Ile
           545                 550                 555
   aca cta cca atg cag gat aac aat act gat gtg tac tgt att cgt tct    1791
   Thr Leu Pro Met Gln Asp Asn Asn Thr Asp Val Tyr Cys Ile Arg Ser
       560                 565                 570
   aac caa ttc tca gtt tat gtt cat tcc act tgc aaa agt tct tta tgg    1839
   Asn Gln Phe Ser Val Tyr Val His Ser Thr Cys Lys Ser Ser Leu Trp
   575                 580                 585                 590
   gac aat att ttt aat caa gac tgc acg gat gtt tta gag gct aca gct    1887
   Asp Asn Ile Phe Asn Gln Asp Cys Thr Asp Val Leu Glu Ala Thr Ala
                   595                 600                 605
   gtt ata aaa act ggt act tgt cct ttc tca ttt gat aaa ttg aac aat    1935
   Val Ile Lys Thr Gly Thr Cys Pro Phe Ser Phe Asp Lys Leu Asn Asn
               610                 615                 620
   tac ttg act ttt aac aag ttc tgt ttg tcg ttg agt cct gtt ggt gct    1983
   Tyr Leu Thr Phe Asn Lys Phe Cys Leu Ser Leu Ser Pro Val Gly Ala
           625                 630                 635
   aat tgc aag ttt gat gtt gct gca cgt aca aga acc aat gag cag gtt    2031
   Asn Cys Lys Phe Asp Val Ala Ala Arg Thr Arg Thr Asn Glu Gln Val
       640                 645                 650
   gtt aga agt cta tat gta ata tat gaa gaa gga gac aac ata gtg ggt    2079
   Val Arg Ser Leu Tyr Val Ile Tyr Glu Glu Gly Asp Asn Ile Val Gly
   655                 660                 665                 670
   gta ccg tct gat aat agc ggt ctg cac gat ttg tct gtg cta cac cta    2127
   Val Pro Ser Asp Asn Ser Gly Leu His Asp Leu Ser Val Leu His Leu
                   675                 680                 685
   gac tcc tgt aca gat tac aat ata tat ggt aga act ggt gtt ggt att    2175
   Asp Ser Cys Thr Asp Tyr Asn Ile Tyr Gly Arg Thr Gly Val Gly Ile
               690                 695                 700
   att aga cga act aac agt acg cta ctt agt ggc tta tat tac aca tca    2223
   Ile Arg Arg Thr Asn Ser Thr Leu Leu Ser Gly Leu Tyr Tyr Thr Ser
           705                 710                 715
   cta tca ggt gat ttg tta ggc ttt aaa aat gtt agt gat ggt gtc att    2271
   Leu Ser Gly Asp Leu Leu Gly Phe Lys Asn Val Ser Asp Gly Val Ile
       720                 725                 730
   tat tct gtg acg cca tgt gat gta agc gca caa gcg gct gtt att gat    2319
   Tyr Ser Val Thr Pro Cys Asp Val Ser Ala Gln Ala Ala Val Ile Asp
   735                 740                 745                 750
   ggt gcc ata gtt gga gct atg act tcc att aac agt gaa ctg tta ggt    2367
   Gly Ala Ile Val Gly Ala Met Thr Ser Ile Asn Ser Glu Leu Leu Gly
                   755                 760                 765
   cta aca cat tgg aca acg aca cct aat ttt tat tac tac tct ata tat     2415
   Leu Thr His Trp Thr Thr Thr Pro Asn Phe Tyr Tyr Tyr Ser Ile Tyr
               770                 775                 780
   aat tac aca agt gag agg act cgt ggc act gca att gac agt aac gat     2463
   Asn Tyr Thr Ser Glu Arg Thr Arg Gly Thr Ala Ile Asp Ser Asn Asp
           785                 790                 795
   gtt gat tgt gaa cct gtc ata acc tat tct aat ata ggt gtt tgt aaa     2511
   Val Asp Cys Glu Pro Val Ile Thr Tyr Ser Asn Ile Gly Val Cys Lys
       800                 805                 810
   aat ggt gct ttg gtt ttt att aac gtc aca cat tct gac gga gac gtg     2559
   Asn Gly Ala Leu Val Phe Ile Asn Val Thr His Ser Asp Gly Asp Val
   815                 820                 825                 830
   caa cca att agc act ggt aat gtc acg ata cct aca aat ttt act ata     2607
   Gln Pro Ile Ser Thr Gly Asn Val Thr Ile Pro Thr Asn Phe Thr Ile
                   835                 840                 845
   tct gtg caa gtt gaa tac atg cag gtt tac act aca cca gta tca ata     2655
   Ser Val Gln Val Glu Tyr Met Gln Val Tyr Thr Thr Pro Val Ser Ile
               850                 855                 860
   gat tgt gca aga tac gtt tgt aat ggt aac cct aga tgt aac aaa ttg     2703
   Asp Cys Ala Arg Tyr Val Cys Asn Gly Asn Pro Arg Cys Asn Lys Leu
           865                 870                 875
   tta aca caa tat gtg tct gca tgt caa act att gaa caa gca ctt gca     2751
   Leu Thr Gln Tyr Val Ser Ala Cys Gln Thr Ile Glu Gln Ala Leu Ala
       880                 885                 890
   atg ggt gcc aga ctt gaa aac atg gag gtt gat tcc atg ttg ttt gtc     2799
   Met Gly Ala Arg Leu Glu Asn Met Glu Val Asp Ser Met Leu Phe Val
   895                 900                 905                 910
   tcg gaa aat gcc ctt aaa ttg gca tct gtt gag gcg ttc aat agt aca     2847
   Ser Glu Asn Ala Leu Lys Leu Ala Ser Val Glu Ala Phe Asn Ser Thr
                   915                 920                 925
   gaa aat tta gat cct att tac aaa gaa tgg cct agc ata ggt ggt tct     2895
   Glu Asn Leu Asp Pro Ile Tyr Lys Glu Trp Pro Ser Ile Gly Gly Ser
               930                 935                 940
   tgg cta gga ggt cta aaa gat ata cta ccg tcc cat aat agc aaa cgt     2943
   Trp Leu Gly Gly Leu Lys Asp Ile Leu Pro Ser His Asn Ser Lys Arg
           945                 950                 955
   aag tat ggt tct gct ata gaa gat ttg ctt ttt gat aaa gtt gta aca     2991
   Lys Tyr Gly Ser Ala Ile Glu Asp Leu Leu Phe Asp Lys Val Val Thr
       960                 965                 970
   tct ggt tta ggt aca gtt gat gaa gat tat aaa cgt tgt act ggt ggt     3039
   Ser Gly Leu Gly Thr Val Asp Glu Asp Tyr Lys Arg Cys Thr Gly Gly
   975                 980                 985                 990
   tac gac ata gca gac ttg gtg tgt gct caa tat tac aat ggc atc atg   3087
   Tyr Asp Ile Ala Asp Leu Val Cys Ala Gln Tyr Tyr Asn Gly Ile Met
                   995                1000                1005
   gtt cta cca ggt gta gct aat gct gac aag atg act atg tac aca gca   3135
   Val Leu Pro Gly Val Ala Asn Ala Asp Lys Met Thr Met Tyr Thr Ala
               1010                1015                1020
   tca ctt gca ggt ggt ata aca tta ggt gca ctt ggt ggt ggc gcc gtg   3183
   Ser Leu Ala Gly Gly Ile Thr Leu Gly Ala Leu Gly Gly Gly Ala Val
           1025                1030                1035
   gct ata cct ttt gca gta gca gta cag gct aga ctt aat tat gtt gct   3231
   Ala Ile Pro Phe Ala Val Ala Val Gln Ala Arg Leu Asn Tyr Val Ala
       1040                1045                1050
   cta caa act gat gta ttg aat aaa aac caa cag atc ctg gct aat gct   3279
   Leu Gln Thr Asp Val Leu Asn Lys Asn Gln Gln Ile Leu Ala Asn Ala
   1055                1060                1065                1070
   ttc aat caa gct att ggt aac att aca cag gct ttt ggt aag gtt aat   3327
   Phe Asn Gln Ala Ile Gly Asn Ile Thr Gln Ala Phe Gly Lys Val Asn
                   1075                1080                1085
   gat gct ata cat caa aca tca caa ggt ctt gcc act gtt gct aaa gcg   3375
   Asp Ala Ile His Gln Thr Ser Gln Gly Leu Ala Thr Val Ala Lys Ala
               1090                1095                1100
   ttg gca aaa gtg caa gat gtt gtc aac aca caa ggg caa gct tta agt   3423
   Leu Ala Lys Val Gln Asp Val Val Asn Thr Gln Gly Gln Ala Leu Ser
           1105                1110                1115
   cac ctt aca gta caa ttg caa aat aat ttt caa gcc att agt agt tct   3471
   His Leu Thr Val Gln Leu Gln Asn Asn Phe Gln Ala Ile Ser Ser Ser
       1120                1125                1130
   att agt gat att tat aac agg ctt gac gaa ctg agt gct gat gca caa   3519
   Ile Ser Asp Ile Tyr Asn Arg Leu Asp Glu Leu Ser Ala Asp Ala Gln
   1135                1140                1145                1150
   gtt gat agg ctg att aca ggt aga ctt aca gca ctt aat gca ttt gtg   3567
   Val Asp Arg Leu Ile Thr Gly Arg Leu Thr Ala Leu Asn Ala Phe Val
                   1155                1160                1165
   tct cag act cta acc aga caa gca gag gtt agg gct agt aga caa ctt   3615
   Ser Gln Thr Leu Thr Arg Gln Ala Glu Val Arg Ala Ser Arg Gln Leu
               1170                1175                1180
   gcc aaa gac aag gtt aat gaa tgt gtt agg tct cag tct cag aga ttc   3663
   Ala Lys Asp Lys Val Asn Glu Cys Val Arg Ser Gln Ser Gln Arg Phe
           1185                1190                1195
   gga ttc tgt ggt aat ggt aca cat ttg ttt tca cta gca aat gca gca   3711
   Gly Phe Cys Gly Asn Gly Thr His Leu Phe Ser Leu Ala Asn Ala Ala
       1200                1205                1210
   cca aat ggc atg att ttc ttt cat aca gta cta tta cca aca gct tat   3759
   Pro Asn Gly Met Ile Phe Phe His Thr Val Leu Leu Pro Thr Ala Tyr
   1215                1220                1225                1230
   gaa act gta aca gct tgg tca ggt att tgt gct tca gat ggc gat cgc   3807
   Glu Thr Val Thr Ala Trp Ser Gly Ile Cys Ala Ser Asp Gly Asp Arg
                   1235                1240                1245
   act ttc gga ctt gtc gtt aaa gat gtg cag ttg acg ttg ttt cgt aat   3855
   Thr Phe Gly Leu Val Val Lys Asp Val Gln Leu Thr Leu Phe Arg Asn
               1250                1255                1260
   cta gat gac aag ttc tat ttg acc ccc aga act atg tat cag cct aga   3903
   Leu Asp Asp Lys Phe Tyr Leu Thr Pro Arg Thr Met Tyr Gln Pro Arg
           1265                1270                1275
   gtt gca act agt tct gat ttt gtt caa att gaa ggg tgt gat gtg ttg   3951
   Val Ala Thr Ser Ser Asp Phe Val Gln Ile Glu Gly Cys Asp Val Leu
       1280                1285                1290
   ttt gtc aac gcg act gta att gat ttg cct agt att ata cct gac tat   3999
   Phe Val Asn Ala Thr Val Ile Asp Leu Pro Ser Ile Ile Pro Asp Tyr
   1295                1300                1305                1310
   att gac att aat caa act gtt caa gac ata tta gaa aat tac aga cca   4047
   Ile Asp Ile Asn Gln Thr Val Gln Asp Ile Leu Glu Asn Tyr Arg Pro
                   1315                1320                1325
   aac tgg act gta cct gaa ttt aca ctt gat att ttc aac gca acc tat   4095
   Asn Trp Thr Val Pro Glu Phe Thr Leu Asp Ile Phe Asn Ala Thr Tyr
               1330                1335                1340
   tta aat ctg act ggt gaa att gat gac tta gag ttt agg tca gaa aag   4143
   Leu Asn Leu Thr Gly Glu Ile Asp Asp Leu Glu Phe Arg Ser Glu Lys
           1345                1350                1355
   cta cat aac act aca gta gaa ctt gcc att ctc att gat aac att aat   4191
   Leu His Asn Thr Thr Val Glu Leu Ala Ile Leu Ile Asp Asn Ile Asn
       1360                1365                1370
   aat aca tta gtc aat ctt gaa tgg ctc aat aga att gaa act tat gta   4239
   Asn Thr Leu Val Asn Leu Glu Trp Leu Asn Arg Ile Glu Thr Tyr Val
   1375                1380                1385                1390
   aaa tgg cct tgg tat gtg tgg cta ctg ata ggt tta gta gta gta ttt   4287
   Lys Trp Pro Trp Tyr Val Trp Leu Leu Ile Gly Leu Val Val Val Phe
                   1395                1400                1405
   tgc ata cca tta ctg cta ttt tgc tgt ttt agc aca ggt tgt tgt gga   4335
   Cys Ile Pro Leu Leu Leu Phe Cys Cys Phe Ser Thr Gly Cys Cys Gly
               1410                1415                1420
   tgc ata ggt tgt tta gga agt tgt tgt cac tct ata tgt agt aga aga   4383
   Cys Ile Gly Cys Leu Gly Ser Cys Cys His Ser Ile Cys Ser Arg Arg
           1425                1430                1435
   caa ttt gaa aat tat gaa cca att gaa aaa gtg cat gtc cac taa       4428
   Gln Phe Glu Asn Tyr Glu Pro Ile Glu Lys Val His Val His
      1440                 1445                 1450
   atttaaagtt aaggatgttg aataaattcc ttaagaacta aacttattag tcattacagg 4488
   tcttgtatgg ac                                                     4500
<210> 2
   <211> 21
   <212> PRT
   <213> coronavirus de la péritonite infectieuse féline (protéine S)
<400> 2
   Gly Gly Ser Trp Leu Gly Gly Leu Lys Asp Ile Leu Pro Ser His Asn
     1               5                  10                  15
   Ser Lys Arg Lys Tyr
               20
<210> 3
   <211> 59
   <212> PRT
   <213> coronavirus de la péritonite infectieuse féline (protéine S)
<400> 3
   His Asn Ser Lys Arg Lys Tyr Gly Ser Ala Ile Glu Asp Leu Leu Phe
     1               5                  10                  15
   Asp Lys Val Val Thr Ser Gly Leu Gly Thr Val Asp Glu Asp Tyr Lys
               20                  25                  30
   Arg Cys Thr Gly Gly Tyr Asp Ile Ala Asp Leu Val Cys Ala Gln Tyr
           35                  40                  45
   Tyr Asn Gly Ile Met Val Leu Pro Gly Val Ala
       50                  55
<210> 4
   <211> 31
   <212> PRT
   <213> coronavirus de la péritonite infectieuse féline (protéine S)
<400> 4
   Met Tyr Gln Pro Arg Val Ala Thr Ser Ser Asp Phe Val Gln Ile Glu
     1               5                  10                  15
   Gly Cys Asp Val Leu Phe Val Asn Ala Thr Val Ile Asp Leu Pro
               20                  25                  30
<210> 5
   <211> 365
   <212> PRT
   <213> coronavirus de la péritonite infectieuse féline (protéine S)
<400> 5
   Gly Gly Ser Trp Leu Gly Gly Leu Lys Asp Ile Leu Pro Ser His Asn
   1               5                   10                  15
   Ser Lys Arg Lys Tyr Gly Ser Ala Ile Glu Asp Leu Leu Phe Asp Lys
               20                  25                  30
   Val Val Thr Ser Gly Leu Gly Thr Val Asp Glu Asp Tyr Lys Arg Cys
           35                  40                  45
   Thr Gly Gly Tyr Asp Ile Ala Asp Leu Val Cys Ala Gln Tyr Tyr Asn
       50                  55                  60
   Gly Ile Met Val Leu Pro Gly Val Ala Asn Ala Asp Lys Met Thr Met
   65                  70                  75                  80
   Tyr Thr Ala Ser Leu Ala Gly Gly Ile Thr Leu Gly Ala Leu Gly Gly
                   85                  90                  95
   Gly Ala Val Ala Ile Pro Phe Ala Val Ala Val Gln Ala Arg Leu Asn
               100                 105                 110
   Tyr Val Ala Leu Gln Thr Asp Val Leu Asn Lys Asn Gln Gln Ile Leu
           115                 120                 125
   Ala Asn Ala Phe Asn Gln Ala Ile Gly Asn Ile Thr Gln Ala Phe Gly
       130                 135                 140
   Lys Val Asn Asp Ala Ile His Gln Thr Ser Gln Gly Leu Ala Thr Val
   145                 150                 155                 160
   Ala Lys Ala Leu Ala Lys Val Gln Asp Val Val Asn Thr Gln Gly Gln
                   165                 170                 175
   Ala Leu Ser His Leu Thr Val Gln Leu Gln Asn Asn Phe Gln Ala Ile
               180                 185                 190
   Ser Ser Ser Ile Ser Asp Ile Tyr Asn Arg Leu Asp Glu Leu Ser Ala
           195                 200                 205
   Asp Ala Gln Val Asp Arg Leu Ile Thr Gly Arg Leu Thr Ala Leu Asn
       210                 215                 220
   Ala Phe Val Ser Gln Thr Leu Thr Arg Gln Ala Glu Val Arg Ala Ser
   225                 230                 235                 240
   Arg Gln Leu Ala Lys Asp Lys Val Asn Glu Cys Val Arg Ser Gln Ser
                   245                 250                 255
   Gln Arg Phe Gly Phe Cys Gly Asn Gly Thr His Leu Phe Ser Leu Ala
               260                 265                 270
   Asn Ala Ala Pro Asn Gly Met Ile Phe Phe His Thr Val Leu Leu Pro
   275 280 285
   Thr Ala Tyr Glu Thr Val Thr Ala Trp Ser Gly Ile Cys Ala Ser Asp
   290 295 300
   Gly Asp Arg Thr Phe Gly Leu Val Val Lys Asp Val Gln Leu Thr Leu
   305 310 315 320
   Phe Arg Asn Leu Asp Asp Lys Phe Tyr Leu Thr Pro Arg Thr Met Tyr
   325 330 335
   Gln Pro Arg Val Ala Thr Ser Ser Asp Phe Val Gln Ile Glu Gly Cys
   340 345 350
   Asp Val Leu Phe Val Asn Ala Thr Val Ile Asp Leu Pro
   355 360 365
<210> 6
   <211> 1452
   <212> PRT
   <213> coronavirus de la péritonite infectieuse féline (protéine S)
<400> 6
   Met Ile Val Leu Val Thr Cys Leu Leu Leu Leu Cys Ser Tyr His Thr
   1 5 10 15
   Val Leu Ser Thr Thr Asn Asn Glu Cys Ile Gln Val Asn Val Thr Gln
   20 25 30
   Leu Ala Gly Asn Glu Asn Leu Ile Arg Asp Phe Leu Phe Ser Asn Phe
   35 40 45
   Lys Glu Glu Gly Ser Val Val Val Gly Gly Tyr Tyr Pro Thr Glu Val
   50 55 60
   Trp Tyr Asn Cys Ser Arg Thr Ala Arg Thr Thr Ala Phe Gln Tyr Phe
   65 70 75 80
   Asn Asn Ile His Ala Phe Tyr Phe Val Met Glu Ala Met Glu Asn Ser
   85 90 95
   Thr Gly Asn Ala Arg Gly Lys Pro Leu Leu Phe His Val His Gly Glu
   100 105 110
   Pro Val Ser Val Ile Ile Ser Ala Tyr Arg Asp Asp Val Gln Gln Arg
   115 120 125
   Pro Leu Leu Lys His Gly Leu Val Cys Ile Thr Lys Asn Arg His Ile
   130 135 140
   Asn Tyr Glu Gln Phe Thr Ser Asn Gln Trp Asn Ser Thr Cys Thr Gly
   145 150 155 160
   Ala Asp Arg Lys Ile Pro Phe Ser Val Ile Pro Thr Asp Asn Gly Thr
   165 170 175
   Lys Ile Tyr Gly Leu Glu Trp Asn Asp Asp Phe Val Thr Ala Tyr Ile
   180 185 190
   Ser Gly Arg Ser Tyr His Leu Asn Ile Asn Thr Asn Trp Phe Asn Asn
   195 200 205
   Val Thr Leu Leu Tyr Ser Arg Ser Ser Thr Ala Thr Trp Glu Tyr Ser
   210 215 220
   Ala Ala Tyr Ala Tyr Gln Gly Val Ser Asn Phe Thr Tyr Tyr Lys Leu
   225 230 235 240
   Asn Asn Thr Asn Gly Leu Lys Thr Tyr Glu Leu Cys Glu Asp Tyr Glu
   245 250 255
   His Cys Thr Gly Tyr Ala Thr Asn Val Phe Ala Pro Thr Ser Gly Gly
   260 265 270
   Tyr Ile Pro Asp Gly Phe Ser Phe Asn Asn Trp Phe Leu Leu Thr Asn
   275 280 285
   Ser Ser Thr Phe Val Ser Gly Arg Phe Val Thr Asn Gln Pro Leu Leu
   290 295 300
   Ile Asn Cys Leu Trp Pro Val Pro Ser Phe Gly Val Ala Ala Gln Glu
   305 310 315 320
   Phe Cys Phe Glu Gly Ala Gln Phe Ser Gln Cys Asn Gly Val Ser Leu
   325 330 335
   Asn Asn Thr Val Asp Val Ile Arg Phe Asn Leu Asn Phe Thr Ala Asp
   340 345 350
   Val Gln Ser Gly Met Gly Ala Thr Val Phe Ser Leu Asn Thr Thr Gly
   355 360 365
   Gly Val Ile Leu Glu Ile Ser Cys Tyr Ser Asp Thr Val Ser Glu Ser
   370 375 380
   Ser Ser Tyr Ser Tyr Gly Glu Ile Pro Phe Gly Ile Thr Asp Gly Pro
   385 390 395 400
   Arg Tyr Cys Tyr Val Leu Tyr Asn Gly Thr Ala Leu Lys Tyr Leu Gly
   405 410 415
   Thr Leu Pro Pro Ser Val Lys Glu Ile Ala Ile Ser Lys Trp Gly His
   420 425 430
   Phe Tyr Ile Asn Gly Tyr Asn Phe Phe Ser Thr Phe Pro Ile Gly Cys
   435 440 445
   Ile Ser Phe Asn Leu Thr Thr Gly Val Ser Gly Ala Phe Trp Thr Ile
   450 455 460
   Ala Tyr Thr Ser Tyr Thr Glu Ala Leu Val Gln Val Glu Asn Thr Ala
   465 470 475 480
   Ile Lys Asn Val Thr Tyr Cys Asn Ser His Ile Asn Asn Ile Lys Cys
                   485                 490                 495
   Ser Gln Leu Thr Ala Asn Leu Asn Asn Gly Phe Tyr Pro Val Ala Ser
               500                 505                 510
   Ser Glu Val Gly Phe Val Asn Lys Ser Val Val Leu Leu Pro Ser Phe
           515                 520                 525
   Phe Thr Tyr Thr Ala Val Asn Ile Thr Ile Asp Leu Gly Met Lys Leu
       530                 535                 540
   Ser Gly Tyr Gly Gln Pro Ile Ala Ser Thr Leu Ser Asn Ile Thr Leu
   545                 550                 555                 560
   Pro Met Gln Asp Asn Asn Thr Asp Val Tyr Cys Ile Arg Ser Asn Gln
                   565                 570                 575
   Phe Ser Val Tyr Val His Ser Thr Cys Lys Ser Ser Leu Trp Asp Asn
               580                 585                 590
   Ile Phe Asn Gln Asp Cys Thr Asp Val Leu Glu Ala Thr Ala Val Ile
           595                 600                 605
   Lys Thr Gly Thr Cys Pro Phe Ser Phe Asp Lys Leu Asn Asn Tyr Leu
       610                 615                 620
   Thr Phe Asn Lys Phe Cys Leu Ser Leu Ser Pro Val Gly Ala Asn Cys
   625                 630                 635                 640
   Lys Phe Asp Val Ala Ala Arg Thr Arg Thr Asn Glu Gln Val Val Arg
                   645                 650                 655
   Ser Leu Tyr Val Ile Tyr Glu Glu Gly Asp Asn Ile Val Gly Val Pro
               660                 665                 670
   Ser Asp Asn Ser Gly Leu His Asp Leu Ser Val Leu His Leu Asp Ser
           675                 680                 685
   Cys Thr Asp Tyr Asn Ile Tyr Gly Arg Thr Gly Val Gly Ile Ile Arg
       690                 695                 700
   Arg Thr Asn Ser Thr Leu Leu Ser Gly Leu Tyr Tyr Thr Ser Leu Ser
   705                 710                 715                 720
   Gly Asp Leu Leu Gly Phe Lys Asn Val Ser Asp Gly Val Ile Tyr Ser
                   725                 730                 735
   Val Thr Pro Cys Asp Val Ser Ala Gln Ala Ala Val Ile Asp Gly Ala
               740                 745                 750
   Ile Val Gly Ala Met Thr Ser Ile Asn Ser Glu Leu Leu Gly Leu Thr
           755                 760                 765
   His Trp Thr Thr Thr Pro Asn Phe Tyr Tyr Tyr Ser Ile Tyr Asn Tyr
       770                 775                 780
   Thr Ser Glu Arg Thr Arg Gly Thr Ala Ile Asp Ser Asn Asp Val Asp
   785 790 795 800
   Cys Glu Pro Val Ile Thr Tyr Ser Asn Ile Gly Val Cys Lys Asn Gly
   805 810 815
   Ala Leu Val Phe Ile Asn Val Thr His Ser Asp Gly Asp Val Gln Pro
   820 825 830
   Ile Ser Thr Gly Asn Val Thr Ile Pro Thr Asn Phe Thr Ile Ser Val
   835 840 845
   Gln Val Glu Tyr Met Gln Val Tyr Thr Thr Pro Val Ser Ile Asp Cys
   850 855 860
   Ala Arg Tyr Val Cys Asn Gly Asn Pro Arg Cys Asn Lys Leu Leu Thr
   865 870 875 880
   Gln Tyr Val Ser Ala Cys Gln Thr Ile Glu Gln Ala Leu Ala Met Gly
   885 890 895
   Ala Arg Leu Glu Asn Met Glu Val Asp Ser Met Leu Phe Val Ser Glu
   900 905 910
   Asn Ala Leu Lys Leu Ala Ser Val Glu Ala Phe Asn Ser Thr Glu Asn
   915 920 925
   Leu Asp Pro Ile Tyr Lys Glu Trp Pro Ser Ile Gly Gly Ser Trp Leu
   930 935 940
   Gly Gly Leu Lys Asp Ile Leu Pro Ser His Asn Ser Lys Arg Lys Tyr
   945 950 955 960
   Gly Ser Ala Ile Glu Asp Leu Leu Phe Asp Lys Val Val Thr Ser Gly
   965 970 975
   Leu Gly Thr Val Asp Glu Asp Tyr Lys Arg Cys Thr Gly Gly Tyr Asp
   980 985 990
   Ile Ala Asp Leu Val Cys Ala Gln Tyr Tyr Asn Gly Ile Met Val Leu
   995 1000 1005
   Pro Gly Val Ala Asn Ala Asp Lys Met Thr Met Tyr Thr Ala Ser Leu
   1010 1015 1020
   Ala Gly Gly Ile Thr Leu Gly Ala Leu Gly Gly Gly Ala Val Ala Ile
   025 1030 1035 1040
   Pro Phe Ala Val Ala Val Gln Ala Arg Leu Asn Tyr Val Ala Leu Gln
   1045 1050 1055
   Thr Asp Val Leu Asn Lys Asn Gln Gln Ile Leu Ala Asn Ala Phe Asn
   1060 1065 1070
   Gln Ala Ile Gly Asn Ile Thr Gln Ala Phe Gly Lys Val Asn Asp Ala
   1075 1080 1085
   Ile His Gln Thr Ser Gln Gly Leu Ala Thr Val Ala Lys Ala Leu Ala
   1090 1095 1100
   Lys Val Gln Asp Val Val Asn Thr Gln Gly Gln Ala Leu Ser His Leu
   105 1110 1115 1120
   Thr Val Gln Leu Gln Asn Asn Phe Gln Ala Ile Ser Ser Ser Ile Ser
   1125 1130 1135
   Asp Ile Tyr Asn Arg Leu Asp Glu Leu Ser Ala Asp Ala Gln Val Asp
   1140 1145 1150
   Arg Leu Ile Thr Gly Arg Leu Thr Ala Leu Asn Ala Phe Val Ser Gln
   1155 1160 1165
   Thr Leu Thr Arg Gln Ala Glu Val Arg Ala Ser Arg Gln Leu Ala Lys
   1170 1175 1180
   Asp Lys Val Asn Glu Cys Val Arg Ser Gln Ser Gln Arg Phe Gly Phe
   185 1190 1195 1200
   Cys Gly Asn Gly Thr His Leu Phe Ser Leu Ala Asn Ala Ala Pro Asn
   1205 1210 1215
   Gly Met Ile Phe Phe His Thr Val Leu Leu Pro Thr Ala Tyr Glu Thr
   1220 1225 1230
   Val Thr Ala Trp Ser Gly Ile Cys Ala Ser Asp Gly Asp Arg Thr Phe
   1235 1240 1245
   Gly Leu Val Val Lys Asp Val Gln Leu Thr Leu Phe Arg Asn Leu Asp
   1250 1255 1260
   Asp Lys Phe Tyr Leu Thr Pro Arg Thr Met Tyr Gln Pro Arg Val Ala
   265 1270 1275 1280
   Thr Ser Ser Asp Phe Val Gln Ile Glu Gly Cys Asp Val Leu Phe Val
   1285 1290 1295
   Asn Ala Thr Val Ile Asp Leu Pro Ser Ile Ile Pro Asp Tyr Ile Asp
   1300 1305 1310
   Ile Asn Gln Thr Val Gln Asp Ile Leu Glu Asn Tyr Arg Pro Asn Trp
   1315 1320 1325
   Thr Val Pro Glu Phe Thr Leu Asp Ile Phe Asn Ala Thr Tyr Leu Asn
   1330 1335 1340
   Leu Thr Gly Glu Ile Asp Asp Leu Glu Phe Arg Ser Glu Lys Leu His
   345 1350 1355 1360
   Asn Thr Thr Val Glu Leu Ala Ile Leu Ile Asp Asn Ile Asn Asn Thr
   1365 1370 1375
   Leu Val Asn Leu Glu Trp Leu Asn Arg Ile Glu Thr Tyr Val Lys Trp
   1380 1385 1390
   Pro Trp Tyr Val Trp Leu Leu Ile Gly Leu Val Val Val Phe Cys Ile
   1395 1400 1405
   Pro Leu Leu Leu Phe Cys Cys Phe Ser Thr Gly Cys Cys Gly Cys Ile
   1410 1415 1420
   Gly Cys Leu Gly Ser Cys Cys His Ser Ile Cys Ser Arg Arg Gln Phe
   425 1430 1435 1440
   Glu Asn Tyr Glu Pro Ile Glu Lys Val His Val His
   1445 1450
<210> 7
   <211> 63
   <212> ADN
   <213> coronavirus de la péritonite infectieuse féline (protéine S)
<400> 7
   ggtggttctt ggctaggagg tctaaaagat atactaccgt cccataatag caaacgtaag 60
   tat 63
<210> 8
   <211> 177
   <212> ADN
   <213> coronavirus de la péritonite infectieuse féline (protéine S)
<400> 8
   cataatagca aacgtaagta tggttctgct atagaagatt tgctttttga taaagttgta 60
   acatctggtt taggtacagt tgatgaagat tataaacgtt gtactggtgg ttacgacata 120
   gcagacttgg tgtgtgctca atattacaat ggcatcatgg ttctaccagg tgtagct 177
<210> 9
   <211> 93
   <212> ADN
   <213> coronavirus de la péritonite infectieuse féline (protéine S)
<400> 9
   atgtatcagc ctagagttgc aactagttct gattttgttc aaattgaagg gtgtgatgtg 60
   ttgtttgtca acgcgactgt aattgatttg cct 93
<210> 10
   <211> 1095
   <212> ADN
   <213> coronavirus de la péritonite infectieuse féline (protéine S)
<400> 10
   ggtggttctt ggctaggagg tctaaaagat atactaccgt cccataatag caaacgtaag 60
   tatggttctg ctatagaaga tttgcttttt gataaagttg taacatctgg tttaggtaca 120
   gttgatgaag attataaacg ttgtactggt ggttacgaca tagcagactt ggtgtgtgct 180
   caatattaca atggcatcat ggttctacca ggtgtagcta atgctgacaa gatgactatg 240
   tacacagcat cacttgcagg tggtataaca ttaggtgcac ttggtggtgg cgccgtggct 300
   ataccttttg cagtagcagt acaggctaga cttaattatg ttgctctaca aactgatgta 360
   ttgaataaaa accaacagat cctggctaat gctttcaatc aagctattgg taacattaca 420
   caggcttttg gtaaggttaa tgatgctata catcaaacat cacaaggtct tgccactgtt 480
   gctaaagcgt tggcaaaagt gcaagatgtt gtcaacacac aagggcaagc tttaagtcac 540
   cttacagtac aattgcaaaa taattttcaa gccattagta gttctattag tgatatttat 600
   aacaggcttg acgaactgag tgctgatgca caagttgata ggctgattac aggtagactt 660
   acagcactta atgcatttgt gtctcagact ctaaccagac aagcagaggt tagggctagt 720
   agacaacttg ccaaagacaa ggttaatgaa tgtgttaggt ctcagtctca gagattcgga 780
   ttctgtggta atggtacaca tttgttttca ctagcaaatg cagcaccaaa tggcatgatt 840
   ttctttcata cagtactatt accaacagct tatgaaactg taacagcttg gtcaggtatt 900
   tgtgcttcag atggcgatcg cactttcgga cttgtcgtta aagatgtgca gttgacgttg 960
   tttcgtaatc tagatgacaa gttctatttg acccccagaa ctatgtatca gcctagagtt 1020
   gcaactagtt ctgattttgt tcaaattgaa gggtgtgatg tgttgtttgt caacgcgact 1080
   gtaattgatt tgcct                                                  1095